(19) Europäisches Patentamt
European Patent Office
Office européen des brevets

(11) EP 2 473 152 B1

(12) **FASCICULE DE BREVET EUROPEEN**

(45) Date de publication et mention de la délivrance du brevet:
**18.03.2020 Bulletin 2020/12**

(21) Numéro de dépôt: **10763794.4**

(22) Date de dépôt: **01.09.2010**

(51) Int Cl.:
*A61K 8/41* (2006.01) *A61K 8/49* (2006.01)
*A61K 8/19* (2006.01) *A61K 8/35* (2006.01)
*A61Q 5/06* (2006.01) *A61Q 5/10* (2006.01)

(86) Numéro de dépôt international:
**PCT/FR2010/051821**

(87) Numéro de publication internationale:
**WO 2011/027077 (10.03.2011 Gazette 2011/10)**

(54) **COMPOSITION COMPRENANT UN COLORANT HYDROPHOBE, UN AGENT ALCALIN ORGANIQUE ET/OU MINÉRAL PARTICULIER, UN COMPOSÉ PARTICULIER (I) ET UN COMPOSÉ ORGANIQUE PARTICULIER (II), ET SON UTILISATION EN COLORATION**

ZUSAMMENSETZUNG MIT EINEM HYDROPHOBEN FARBSTOFF, EINEM SPEZIFISCHEN MINERAL UND/ODER ORGANISCHEN ALKALISCHEN STOFF, EINER SPEZIFISCHEN VERBINDUNG (I) UND EINER SPEZIFISCHEN ORGANISCHEN VERBINDUNG (II) SOWIE IHRE VERWENDUNG ZUR FÄRBUNG

COMPOSITION COMPRISING A HYDROPHOBIC DYE, A SPECIFIC MINERAL AND/OR ORGANIC ALKALINE AGENT, A SPECIFIC COMPOUND (I) AND A SPECIFIC ORGANIC COMPOUND (II), AND USE THEREOF IN DYEING

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité:
**02.09.2009 FR 0955980**
**02.09.2009 FR 0955982**
**02.09.2009 FR 0955979**
**14.09.2009 US 242130 P**
**15.09.2009 US 242437 P**
**15.09.2009 US 242440 P**
**18.12.2009 FR 0959226**
**22.01.2010 US 297439 P**

(43) Date de publication de la demande:
**11.07.2012 Bulletin 2012/28**

(73) Titulaire: **L'Oréal**
**75008 Paris (FR)**

(72) Inventeurs:
- **LALLEMAN, Boris**
**F-75004 Paris (FR)**
- **LAGRANGE, Alain**
**F-77700 Coupvray (FR)**

(74) Mandataire: **Dossmann, Gérard**
**Casalonga & Partners**
**Bayerstrasse 71-73**
**80335 München (DE)**

(56) Documents cités:
**EP-A1- 0 357 548**
**EP-A1- 1 627 626**
**DE-A1- 4 434 494**
**DE-A1-102004 014 764**
**DE-A1-102004 052 480**
**DE-A1-102007 042 286**
**FR-A1- 2 771 286**
**US-A1- 2008 229 520**

Remarques:
Le dossier contient des informations techniques présentées postérieurement au dépôt de la demande et ne figurant pas dans le présent fascicule.

EP 2 473 152 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention se rapporte à une composition de coloration des fibres kératiniques humaines, comprenant au moins un colorant hydrophobe, au moins un agent alcalin organique et/ou au moins une base minérale particulière, au moins un composé particulier (I) et au moins un composé organique particulier (II), ainsi qu'à un procédé de coloration.

On connaît deux grands modes de coloration des fibres kératiniques humaines, et en particulier des cheveux.

Le premier, appelé coloration d'oxydation ou permanente, consiste à mettre en œuvre un ou plusieurs précurseurs de colorant d'oxydation, plus particulièrement une ou plusieurs bases d'oxydation, éventuellement associées à un ou plusieurs coupleurs.

Habituellement, des bases d'oxydation sont choisies parmi les ortho- ou para-phénylènediamines, les ortho- ou para-aminophénols ainsi que des composés hétérocycliques. Ces bases d'oxydation sont des composés incolores ou faiblement colorés qui, associés à des produits oxydants, permettent d'accéder, par un processus de condensation oxydative, à des espèces colorées qui restent piégées à l'intérieur de la fibre.

Bien souvent, on fait varier les nuances obtenues avec ces bases d'oxydation en les associant à un ou plusieurs coupleurs, ces derniers étant choisis notamment parmi les métadiamines aromatiques, les méta-aminophénols, les métadiphénols et certains composés hétérocycliques, tels que des composés indoliques.

La variété des molécules mises en jeu au niveau des bases d'oxydation et des coupleurs permet l'obtention d'une riche palette de couleurs.

Les procédés de coloration d'oxydation sont généralement mis en œuvre en appliquant sur les fibres kératiniques les précurseurs de colorants d'oxydation (une ou plusieurs bases d'oxydation, éventuellement associées à un ou plusieurs coupleurs) en présence d'un agent oxydant, tel que notamment le peroxyde d'hydrogène (ou « eau oxygénée »), qui est mélangé à la composition colorante juste avant son emploi.

Les colorations résultantes sont généralement puissantes et présentent une bonne ténacité notamment aux shampooings.

Cependant, les conditions de mise en œuvre sont susceptibles d'entraîner une dégradation des fibres kératiniques. A la longue, ces dernières sont plus ou moins dégradées et ont tendance à devenir rêches, ternes, cassantes et difficiles à coiffer, notamment dans le cas de colorations répétées.

Le deuxième mode de coloration, appelé coloration directe ou semi-permanente, comprend l'application de colorants directs, qui sont des molécules ayant une affinité pour les fibres et colorantes, même en l'absence d'agent oxydant ajouté dans les compositions les contenant. Etant donnée la nature des molécules employées, celles-ci restent plutôt en surface de la fibre et pénètrent relativement peu à l'intérieur de la fibre, comparées aux petites molécules de précurseurs de colorants d'oxydation.

Les colorants directs généralement employés sont choisis parmi les colorants directs nitrés benzéniques, anthraquinoniques, nitropyridiniques, azoïques, méthiniques, azométhiniques, xanthéniques, acridiniques, aziniques ou triarylméthaniques. Les espèces chimiques mises en œuvre peuvent être non ioniques, anioniques (colorants acides) ou cationiques (colorants basiques). Les colorants directs peuvent également être des colorants naturels.

La plupart des colorants directs mis en œuvre ont une solubilité en milieu aqueux suffisante et il existe maintenant de nombreux supports de coloration adaptés pour les mettre en oeuvre.

Ces compositions, contenant un ou plusieurs colorants directs, sont appliquées sur les fibres kératiniques pendant un temps nécessaire à l'obtention de la coloration désirée, puis rincées.

Cependant, les colorations qui en résultent sont des colorations particulièrement chromatiques mais temporaires ou semi-permanentes car la nature des interactions, qui lient les colorants directs à la fibre kératinique, et leur désorption de la surface et/ou du cœur de la fibre, sont responsables de leur faible puissance tinctoriale et de leur mauvaise tenue aux lavages ou à la lumière.

Ainsi, les procédés connus de coloration capillaire, que ce soit dans le domaine de la coloration directe ou de la coloration d'oxydation, sont susceptibles d'être améliorés.

Notamment, il existe un besoin de fournir des procédés de coloration des fibres kératiniques humaines qui permettent de respecter la nature ces fibres, et en particulier d'éviter leur dégradation, tout en offrant des teintures puissantes, et résistantes aux agents extérieurs, en particulier aux lavages, à la transpiration, à la lumière, aux rayonnements UV, aux intempéries, aux frottements et aux traitements chimiques, tels que le traitement d'ondulations permanentes.

Ce besoin se fait en particulier ressentir avec les colorants bleus, dont la ténacité à la lumière et aux rayonnements UV est généralement peu satisfaisante.

Pour colorer efficacement les fibres kératiniques, la plupart des colorants directs hydrophobes doivent être employés en présence de solvants particuliers, dont le rôle est de les vectoriser dans la fibre. Parmi les solvants cosmétiques connus pour cet effet, on utilise fréquemment les solvants aromatiques. On peut citer plus particulièrement l'alcool benzylique, le benzyloxyéthanol ou encore le phénoxyéthanol.

Cependant, la présence des solvants aromatiques, très peu solubles en milieu aqueux, nécessite l'emploi de

quantités importantes de co-solvants, habituellement l'éthanol, pour les rendre compatibles avec les formulations tinctoriales classiques.

Par ailleurs, même dans ces conditions, l'intensité des colorations obtenues demeure insuffisante ou la sélectivité demeure trop élevée.

En outre, des agents réducteurs tels que les hydrosulfites sont parfois employés dans le domaine textile, afin de réduire les colorants hydrophobes réductibles. Cependant, ces composés sont nocifs pour l'environnement, et contaminent notamment de manière importante les eaux usées. D'autres part, ils dégagent des odeurs très inconfortantes, qui les rendent peu appropriés à une utilisation dans le domaine cosmétique.

Il existe donc un besoin de fournir de nouvelles compositions à base de colorants hydrophobes pour la teinture des cheveux humains, qui soient plus efficaces, tout en étant compatibles avec les colorants hydrophobes, et qui respectent tant l'environnement que la nature des cheveux. Ces compositions doivent également permettre d'obtenir des teintures puissantes, peu sélectives et résistantes.

La demande de brevet FR 2 771 286 au nom de la Demanderesse décrit, d'une manière générale, l'utilisation de composés de type indigoïde dans des compositions cosmétiques, et plus particulièrement dans des compositions de maquillage telles que des rouges à lèvres, des fonds de teint, des fards à joues ou à paupières, des poudres libres ou compactes, des eye-liners, des mascaras, des vernis à ongles.

La demanderesse a découvert de manière surprenante que les problèmes exposés ci-avant pouvaient être résolus par une composition tinctoriale comprenant un ou plusieurs colorants directs hydrophobes de logP supérieur ou égal à 2, un ou plusieurs agents alcalins organiques et/ou une ou plusieurs bases minérales particulières, un ou plusieurs composés (I) particuliers et un ou plusieurs composés organiques (II) particuliers.

L'invention a donc pour objet une composition tinctoriale comprenant :

- un ou plusieurs colorants directs hydrophobes de logP supérieur ou égal à 2, choisis parmi les colorants indigoïdes,
- un ou plusieurs agents alcalins organiques, et/ou une ou plusieurs bases minérales choisies parmi les carbonates, les hydrogénocarbonates, l'hydroxyde de sodium, l'hydroxyde de potassium, et leurs mélanges,
- un ou plusieurs composés (I) comprenant dans leur structure un enchaînement :

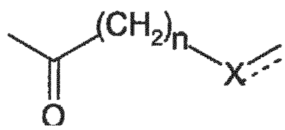

avec n désignant un nombre entier allant de 0 à 4, et 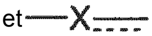 désignant un groupement :

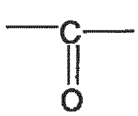 ou 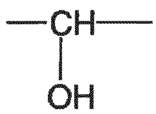 ou 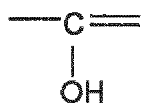 ,

et
- un ou plusieurs composés organiques (II) présentant une valeur du paramètre de solubilité δH de Hansen compris entre 5 et 15 Mpa$^{1/2}$ et de poids moléculaire inférieur à 250 g/mol.

La valeur du logP représente de façon classique le coefficient de partage du colorant entre l'octanol et l'eau. Le logP peut être calculé selon la méthode décrite dans l'article de Meylan et Howard « Atom / Fragment contribution method for estimating octanol-water partition coefficient », J. Pharm. Sci. 84 :83-92, 1995. Cette valeur peut aussi être calculée à partir de nombreux logiciels disponibles sur le marché qui déterminent le logP en fonction de la structure d'une molécule. A titre d'exemple, on peut citer le logiciel Epiwin de l'agence de l'environnement des Etats-Unis.

Il est à noter que la valeur du logP est usuellement donnée pour une température de 25°C et à pression atmosphérique (760 mm Hg, soit 1,013.10$^5$ Pa).

Le ou les colorants directs hydrophobes présents dans la composition selon l'invention sont choisis parmi les colorants indigoïdes.

Les colorants indigoïdes utilisés dans la composition selon l'invention peuvent être choisis parmi :

| Nom | Principes tinctoriaux |
|---|---|
| Indigo | 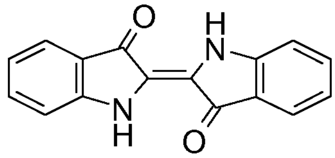 |
| Isoindigo | 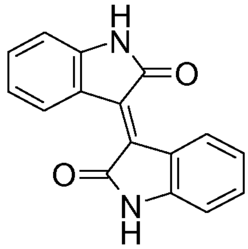 |
| Indirubine | 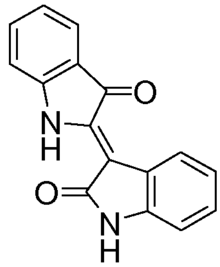 |
| Isoindirubine | 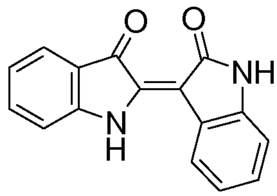 |
| 4,4'-dibromo indigo | 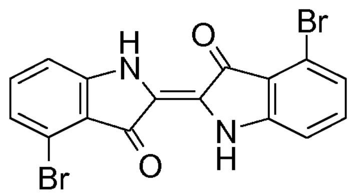 |
| 6,6'-dibromo indigo | 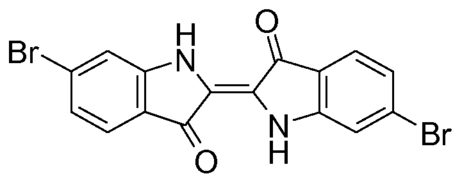 |
| 5,5'-dibromo indigo | 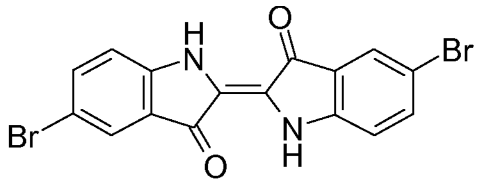 |

(suite)

| Nom | Principes tinctoriaux |
|---|---|
| cis-6,6'-dibromo indigo | 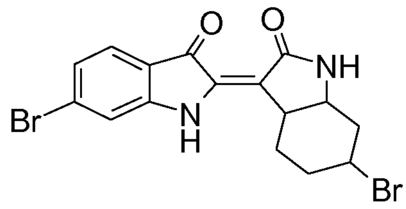 |
| 5,5',7,7'-tétrabromo indigo | 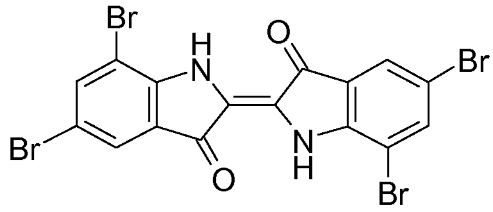 |
| 4,4',7,7'-tétrachloro indigo | 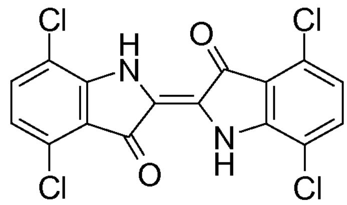 |
| 3H-Indol-3-one, 1,2-dihydro-2-(3-oxobenzo[b]thien-2 (3H)-ylidène) | 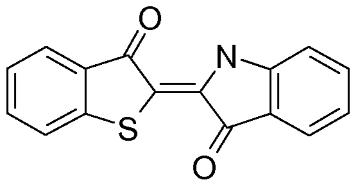 |
| Thioindigo (autres noms:<br>C.I. 73300<br>C.I. Disperse Red 364<br>C.I. Solvent Red 242<br>C.I. Vat Red 41<br>Ciba Pink B<br>Disperse Red 364) | 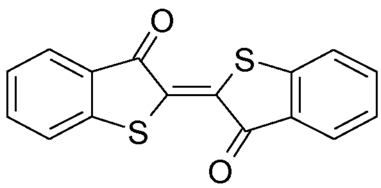 |
| Vat Red 1<br>(Oralith) | 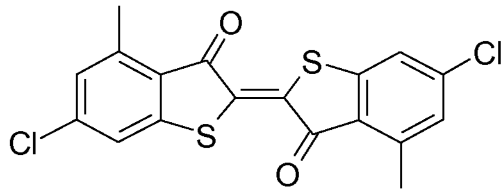 |
| Cis-Thioindigo<br>(Benzo[b]thiophen-3(2H)-one, 2-(3-oxobenzo[b]thien-2 (3H)-ylidène)-,(2Z)) | 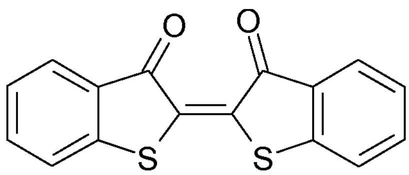 |

(suite)

| Nom | Principes tinctoriaux |
|---|---|
| 6,6'-dichloro-4,4'-diméthylindigo | 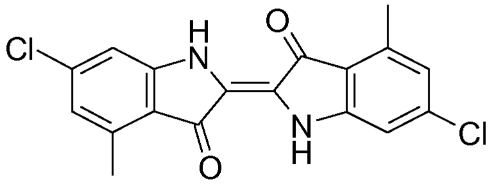 |
| 5,5'-dichloro-7,7'-diméthylindigo | 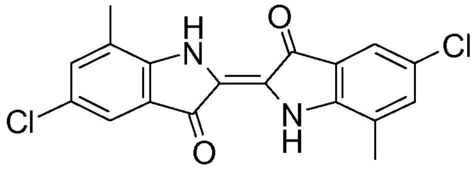 |
| 4,4',7,7'-tétraméthylindigo | 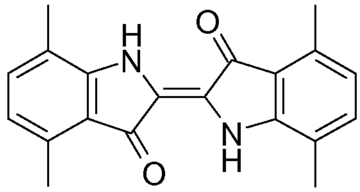 |
| Thioindigo Scarlet R (autre noms: 2H-Indol-2-one, 1,3-dihydro-3-(3-oxobenzo[b] thien-2(3H)-ylidène; C.I. 73635) | 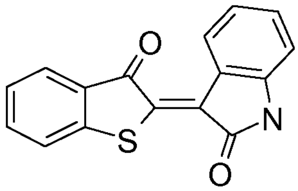 |
| 2H-Indol-2-one, 1,3-dihydro-3-(3-oxobenzo[b]thien-2 (3H)-ylidène)-, (3E)- | 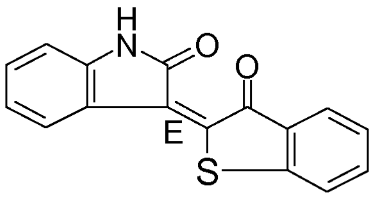 |
| Thioindirubine (Benzo[b]thiophen-3(2H)-one, 2-(2-oxobenzo[b]thiophen-3 (2H)-ylidène)) | 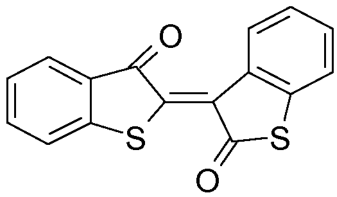 |
| 2H-Indol-2-one, 1,3-dihydro-3-(2-oxobenzo[b]thiophen-3 (2H)-ylidène)- | 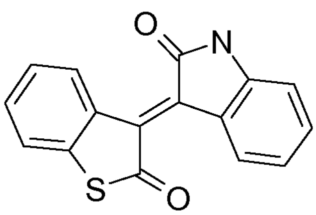 |

(suite)

| Nom | Principes tinctoriaux |
|---|---|
| Benzo[b]thiophen-2(3H)-one, 3-(2-oxobenzo[b]thiophen-3 (2H)-ylidène) | 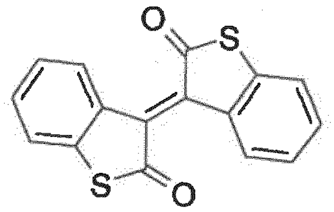 |

On peut aussi utiliser, en tant que colorants directs hydrophobes de logP supérieur ou égal à 2, les colorants directs indigoïdes suivants : le leucoindigo, le leucoisoindigo, le leucoindirubine, le leucoisoindirubine.

Les colorants directs de logP supérieur ou égal à 2 sont choisis parmi les colorants indigoïdes.

Dans les formules (III) et (IV) ci-avant, par atome d'halogène, on désigne en particulier les atomes de chlore, de brome, d'iode et de fluor.

De préférence, les radicaux $R_1$ et $R'_1$, identiques ou différents, représentent un atome d'hydrogène, un radical alkyle en $C_1$ à $C_{10}$ ou un radical alkyloxy en $C_1$ à $C_{10}$, ces radicaux pouvant être substitués ou non par un ou plusieurs atomes d'halogène et/ou par un ou plusieurs groupements hydroxyle.

De manière particulièrement préférée, les radicaux $R_1$ et $R'_1$, identiques ou différents, représentent un atome d'hydrogène ou un radical alkyloxy en $C_1$ à $C_8$.

De préférence, les radicaux $R_2$, $R'_2$, $R_3$, $R'_3$, $R_4$, $R'_4$, $R_5$ et $R'_5$, identiques ou différents, représentent un atome d'hydrogène, un atome d'halogène, un radical hydroxyle, un radical alkyle ou alkyloxy en $C_1$ à $C_{10}$, ces radicaux pouvant être substitués ou non par un ou plusieurs atomes d'halogène et/ou par un ou plusieurs groupements hydroxyle.

De manière particulièrement préférée, les radicaux $R_2$, $R'_2$, $R_3$, $R'_3$, $R_4$, $R'_4$, $R_5$, $R'_5$, identiques ou différents, représentent un atome d'hydrogène, un radical hydroxyle, un radical alkyle en $C_1$ à $C_8$, un radical alkyloxy en $C_1$ à $C_8$.

Parmi les composés de formule (III) susceptibles d'être utilisés dans les compositions selon l'invention, sont plus particulièrement préférés les composés suivants :

| Nom | Structure |
|---|---|
| Indigo Russig's Blue | 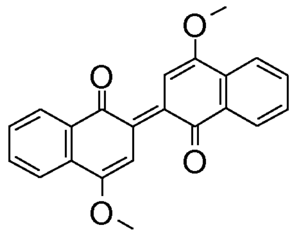 |
| Diosindigo A | 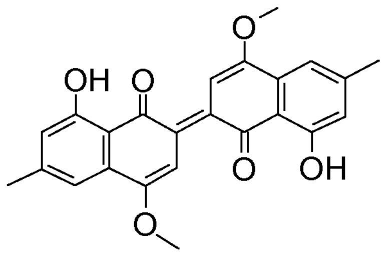 |
| Diosindigo B | 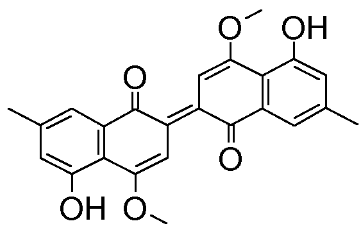 |

(suite)

| Nom | Structure |
|---|---|
| 4,4'-diéthoxy-2,2'-binaphtylidène-1,1'-dione | 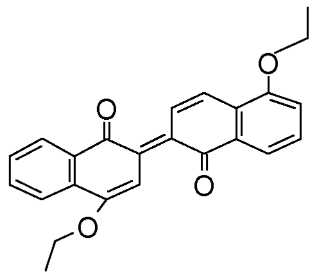 |
| 4,4'-bis(hexyloxy)-1H, 14H-2,2'-binaphtalène-1, 1'-dione | 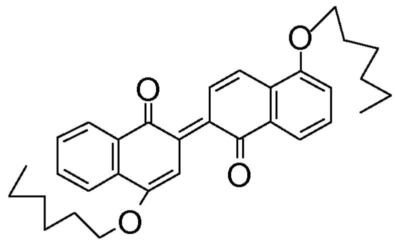 |

Parmi les composés de formule (II) susceptibles d'être utilisés dans les compositions selon l'invention, sont plus particulièrement préférés les composés suivants :

| Nom | Structure |
|---|---|
| mamegakinone | 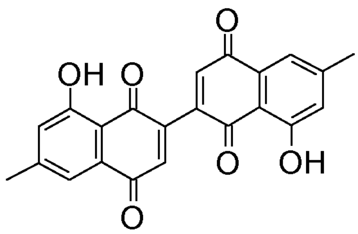 |
| biramentaceone | 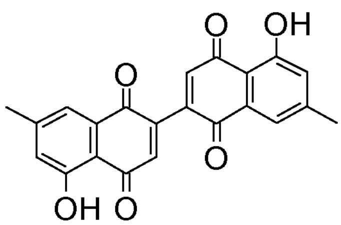 |

Le ou les colorants directs hydrophobes de logP supérieur ou égal à 2 représentent généralement de 0,001 à 20% en poids, de préférence de 0,01 à 20% en poids, préférentiellement de 0,01 à 10 % en poids, encore plus préférentiellement de 0,05 à 10% en poids, mieux de 0,1 à 5% en poids du poids total de la composition cosmétique.

Comme expliqué précédemment, la composition selon l'invention comprend également un ou plusieurs composés particuliers (I).

De préférence, le nombre entier n désigne 0, 1 ou 2.

De préférence, le ou les composés (I) présentent dans leur structure un enchaînement :

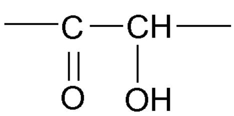 (A) ou 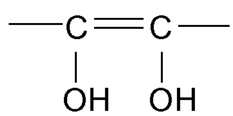 (B),

(B) étant la forme tautomère de (A).

Le ou les composés (I) répondent plus préférentiellement à la formule (C) suivante :

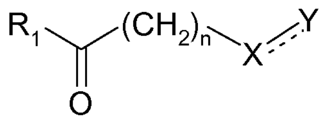

(C)

avec :

n désignant un nombre entier allant de 0 à 4, de préférence de 0 à 2, et 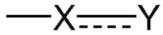 désignant un groupement :

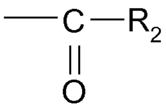 ou 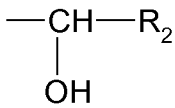 ou 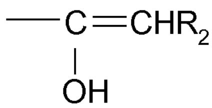 ,

$R_1$ et $R_2$ représentant, indépendamment l'un de l'autre, un atome d'hydrogène ; un radical phényle substitué ou non substitué ; un radical hydroxy ; un radical alcoxy en $C_1$-$C_4$ ; un radical alkyle en $C_1$-$C_4$ linéaire ou ramifié, non substitué ou substitué par un ou deux radicaux choisis parmi les radicaux -OR', -C(O)R", -COOR''', avec R', R" et R''' représentant, indépendamment les uns des autres, un atome d'hydrogène, un radical alkyle en $C_1$-$C_4$ linéaire ou ramifié, ou

$R_1$ et $R_2$ formant, avec la chaîne carbonée à laquelle ils sont attachés, un cycle non aromatique carboné à 5 ou 6 chaînons non substitué.

Le ou les composés particuliers (I) sont choisis de préférence parmi les composés suivants :

| | |
|---|---|
| Hydroxyacétone | 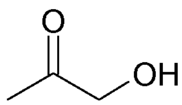 |
| Acétoïne | 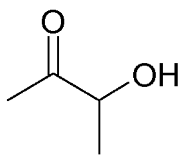 |
| Glutaroïne | 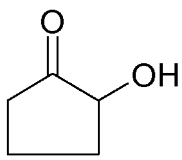 |
| Adipoïne | 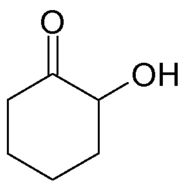 |
| dihydroxyacétone | 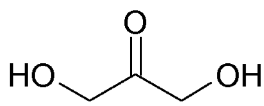 |
| Glycol aldéhyde | 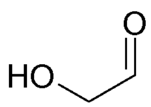 |

(suite)

| | |
|---|---|
| Benzoïne | 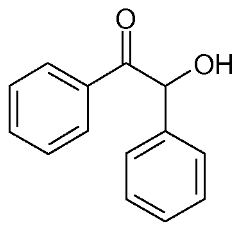 |
| 2,3-dihydroxyacryl-aldéhyde | 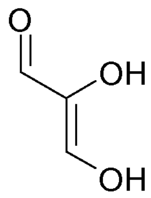 |
| Cyclopentane dione | 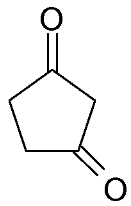 |
| Acétonyl acétone | 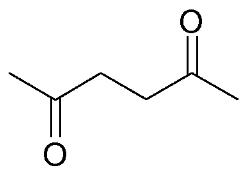 |
| Acétyl acétone | 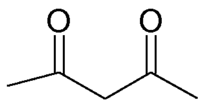 |
| diacétyle | 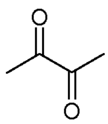 |
| dipropionyle | 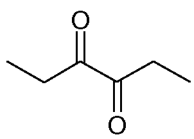 |
| acide 2-cétoglutarique | 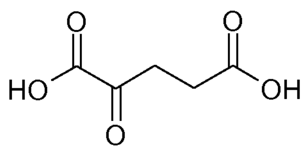 |
| acide 3-cétoglutarique | 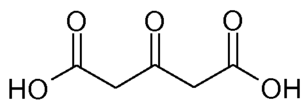 |
| Acide pyruvique | 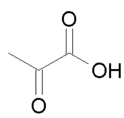 |

(suite)

| | |
|---|---|
| Acide lévulinique | 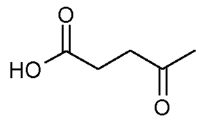 |
| Acide acétoacétique | 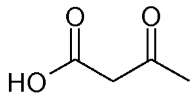 |
| Acide propionylacétique | 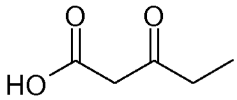 |
| Acide acétonyl malonique | 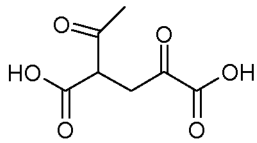 |
| Méthyl acéto acétate | 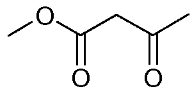 |
| Ethyl acéto acétate | 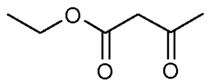 |
| Acide acétopyruvique | 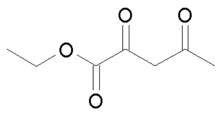 |
| Cyclohexane dione | 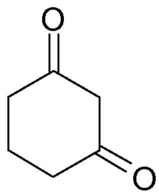 |

Le ou les composés (I) préférés sont les composés α-hydroxyacétoniques, tels que l'hydroxyacétone, l'acétoïne, la glutaroïne, l'adipoïne, la benzoïne, la dihydroxyacétone, le glycol aldéhyde, et le 2,3-dihydroxyacryl-aldéhyde.

Le ou les composés (I) représentent généralement de 0,01 à 20% en poids, de préférence de 0,05 à 10% en poids, mieux de 0,1 à 5% en poids du poids total de la composition cosmétique.

La composition selon l'invention comprend également un ou plusieurs agents alcalins organiques, et/ou une ou plusieurs bases minérales choisies parmi les carbonates, les hydrogénocarbonates, l'hydroxyde de sodium, l'hydroxyde de potassium, et leurs mélanges, et de préférence parmi les agents alcalins organiques, et/ou une ou plusieurs bases minérales choisies parmi les carbonates, les hydrogénocarbonates, et leurs mélanges.

Par « agent alcalin organique », on entend au sens de l'invention un composé organique qui, par sa présence dans la composition, augmente le pH de la composition d'au moins 0,05 unité de pH et de préférence d'au moins 0,1 unité de pH.

De préférence, le ou les agents alcalins organiques de l'invention ont un $pK_b$ à 25°C allant de 1 à 12.

De manière connue en soi, pour un agent alcalin, on désigne par « $pK_b$ » la valeur correspondant à -log($K_b$), avec $K_b$ désignant la constante de dissociation dans l'eau de cet agent alcalin. Lorsque l'agent alcalin organique comprend plus d'une fonction basique, il est à noter que le $pK_b$ selon l'invention correspond à la fonction de basicité la plus élevée.

De préférence, le ou les agents alcalins organiques selon l'invention présentent un $pK_b$ à 25°C allant de 1 à 10, et plus préférentiellement de 2 à 6.

De manière plus préférée, le ou les agents alcalins organiques selon l'invention sont choisis parmi les amines

organiques présentant un $pK_b$ à 25°C tel que défini ci-avant.

Ainsi, selon une première variante, le ou les agents alcalins organiques selon l'invention sont choisis parmi les amines comprenant une fonction amine primaire, secondaire ou tertiaire, et un ou plusieurs groupements alkyle, linéaires ou ramifiés, en $C_1$-$C_8$ porteurs d'un ou plusieurs radicaux hydroxyle.

Conviennent, en particulier, les amines organiques choisies parmi les alcanolamines, telles que les mono-, di- ou tri-alcanolamines, comprenant un à trois radicaux hydroxyalkyle, identiques ou non, en $C_1$-$C_4$.

Parmi ce type de composés, on peut citer la monoéthanolamine, la diéthanolamine, la triéthanolamine, la monoisopropanolamine, la diisopropanolamine, la N-diméthylaminoéthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol, le tris-hydroxyméthylaminométhane.

Selon une deuxième variante, le ou les agents alcalins organiques selon l'invention sont choisis parmi les amines organiques de formule suivante :

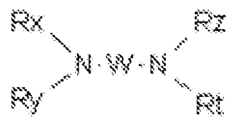

dans laquelle W est un reste alkylène en $C_1$-$C_6$, éventuellement substitué par un groupement hydroxyle ou un radical alkyle en $C_1$-$C_6$, Rx, Ry, Rz et Rt, identiques ou différents, représentent un atome d'hydrogène, un radical alkyle en $C_1$-$C_6$ ou hydroxyalkyle en $C_1$-$C_6$, aminoalkyle en $C_1$-$C_6$.

On peut citer, à titre d'exemple de telles amines, le 1,3-diaminopropane, le 1,3-diamino-2-propanol, la spermine, la spermidine.

Selon une troisième variante, le ou les agents alcalins organiques selon l'invention sont choisis parmi les acides aminés.

Plus particulièrement, les acides aminés utilisables sont d'origine naturelle ou de synthèse, sous leur forme L, D, ou racémique et comportent au moins une fonction acide choisie plus particulièrement parmi les fonctions acides carboxyliques, sulfoniques, phosphoniques ou phosphoriques. Les acides aminés peuvent se trouver sous forme neutre ou ionique.

A titre d'acides aminés utilisables dans la présente invention, on peut notamment citer l'acide aspartique, l'acide glutamique, l'alanine, l'arginine, l'ornithine, la citrulline, l'asparagine, la carnitine, la cystéine, la glutamine, la glycine, l'histidine, la lysine, l'isoleucine, la leucine, la méthionine, la N-phénylalanine, la proline, la serine, la taurine la thréonine, le tryptophane, la tyrosine et la valine.

Les acides aminés préférés sont des acides aminés basiques comprenant une fonction amine supplémentaire éventuellement incluse dans un cycle ou dans une fonction uréido.

De tels acides aminés basiques sont choisis, de préférence, parmi ceux répondant à la formule (V) suivante :

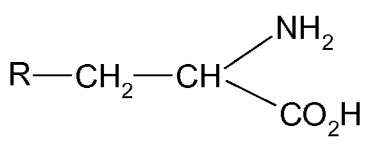
(V)

où R désigne un groupe choisi parmi :

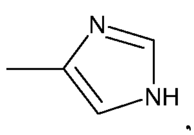

$-(CH_2)_3NH_2$,
$-(CH_2)_2NH_2$,
$-(CH_2)_2NHCONH_2$,

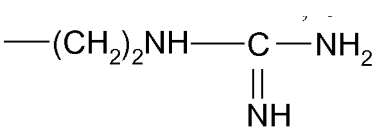

Les composés correspondants à la formule (V) sont l'histidine, la lysine, l'arginine, l'ornithine, la citrulline.

Selon une quatrième variante, le ou les agents alcalins organiques selon l'invention sont choisis parmi les amines organiques de type hétérocyclique. On peut en particulier citer, outre l'histidine déjà mentionnée dans les acides aminés, la pyridine, la pipéridine, l'imidazole, le triazole, le tétrazole, le benzimidazole.

Selon une cinquième variante, le ou les agents alcalins organiques selon l'invention sont choisis parmi les dipeptides d'acides aminés. A titre de dipeptides d'acides aminés utilisables dans la présente invention, on peut notamment citer la carnosine, l'anserine et la baleine.

Selon une sixième variante, le ou les agents alcalins organiques selon l'invention sont choisis parmi les composés comportant une fonction guanidine. Ainsi, l'agent alcalin organique peut être choisi parmi la guanidine, l'arginine déjà mentionnée à titre d'acide aminé, la créatine, la créatinine, la 1,1-diméthylguanidine, la 1,1-diéthylguanidine, la glycocyamine, la metformine, l'agmatine, la n-amidinoalanine, l'acide 3-guanidinopropionique, l'acide 4-guanidinobutyrique et l'acide 2-([amino(imino)méthyl]amino)éthane-1-sulfonique.

De préférence, le ou les agents alcalins organiques sont choisis parmi les alcanolamines, les acides aminés basiques, les composés comportant une fonction guanidine, et leurs mélanges.

De manière encore plus préférée, le ou les agents alcalins organiques sont choisis parmi les alcanolamines, en particulier la monoéthanolamine, la diéthanolamine et la triéthanolamine.

Par « carbonate », on désigne de manière connue en soi un sel contenant l'anion $CO_3^{2-}$.

Par « hydrogénocarbonate», également dénommé bicarbonate, on désigne de manière connue en soi un sel contenant l'anion $HCO_3^-$.

De préférence, la ou les bases minérales sont choisies parmi les carbonates de métaux alcalins, les hydrogénocarbonates de métaux alcalins, et leurs mélanges.

Les métaux alcalins préférés sont le lithium, le sodium et le potassium.

Selon un mode de réalisation particulièrement préféré la ou les bases minérales sont choisies parmi le carbonate de sodium, le carbonate de potassium, l'hydrogénocarbonate de sodium, l'hydrogénocarbonate de potassium, et leurs mélanges.

Le ou les agents alcalins organiques et/ou la ou les bases minérales représentent de 0,1 à 20% en poids, de préférence de 0,5 à 10% en poids, et plus préférentiellement de 1 à 5% en poids, par rapport au poids total de la composition.

La composition selon l'invention comprend également un ou plusieurs composés organiques (II) présentant une valeur du paramètre de solubilité $\delta H$ de Hansen compris entre 5 et 15 $Mpa^{1/2}$, et mieux entre 8 et 15 $Mpa^{1/2}$.

De préférence, ces composés sont liquides à la température de 25°C et à pression atmosphérique (760 mm Hg, soit $1{,}013.10^5$ Pa).

Le ou les composés organiques présentant une valeur du paramètre de solubilité $\delta H$ de Hansen, telle que définie précédemment, sont par exemple décrit dans l'ouvrage de référence «Hansen solubility parameters A user's handbook, Charles M.HANSEN », CRC Press, 2000, pages 167 à 185, ou bien dans l'ouvrage « Handbook of Solubility Parameters and other cohesion parameters », CRC, Press, pages 95 à 121 et pages 177 à 185.

Cette valeur du paramètre de solubilité $\delta H$ est liée à la formation de liaisons hydrogènes.

En particulier, l'ouvrage « Handbook of Solubility Parameters and other cohesion parameters », CRC Press, pages 95 à 121 et pages 177 à 185, donne l'équation $\delta H = (\Sigma\text{-}{}^zU_h/V)^{1/2}$

où,

${}^zU_h$ (en $J.mol^{-1}$) décrit les contributions du groupe fonctionnel considéré dans les paramètres de solubilité lié aux liaisons hydrogènes (valeurs en table 14, page 183), ce paramètre ${}^zU_h$ étant également décrit dans l'ouvrage « The relation between surface tension and solubility parameter in liquids », Bagda, E, Farbe Lack, 84, 212, 1978 ;

et V est le volume de la molécule.

Il est à noter que la valeur du paramètre de solubilité $\delta H$ est usuellement donnée pour une température de 25°C et à pression atmosphérique (760 mm de Hg, soit $1{,}013.10^5$ Pa).

Le ou lesdits composés organiques (II) peuvent être choisis parmi les alcanols, les esters aliphatiques, les éthers, les alcools aromatiques, les alcools alkylaryle, les acides aromatiques, les acides aliphatiques, les carbonates d'alkylène tels que le carbonate de propylène, les lactones comme la ybutyrolactone, et leurs mélanges.

De préférence, le ou lesdits composés organiques (II) sont choisis parmi les alcanols, les esters aliphatiques, les éthers, les alcools aromatiques, les alcools alkylaryle, les acides aromatiques, les acides aliphatiques, et leurs mélanges.

Encore plus préférentiellement, le ou lesdits composés organiques (II) sont choisis parmi le 1-octanol, le 1-décanol, le tridécyl alcool, le dipropylène glycol monométhyléther acétate, le dipropylène glycol méthyléther, le tripropylène glycol méthyléther, le propylène glycol n-butyl éther, le propylène glycol n-propyl éther, le monométhyléther de propylèneglycol, le monoéthyléther et le monométhyléther du diéthylèneglycol, le 3-phényl-1-propanol, le 2-phényl-1-propanol, l'alcool benzylique, le benzyloxyéthanol, le phénoxyéthanol, et les mélanges de ces composés.

Le composé organique (II) est de préférence choisi parmi les alcools aromatiques, les alcools alkylaryle et

encore plus préférentiellement l'alcool benzylique.

Le ou les composés organiques (II), lorsqu'ils sont présents, représentent généralement de 0,1 à 20%, de préférence de 1 à 5%, en poids du poids total de la composition.

La composition selon l'invention peut également comprendre un ou plusieurs colorants additionnels. En particulier, la composition selon l'invention peut également comprendre au moins un colorant additionnel, différents des colorants à caractère hydrophobe de logP supérieur ou égal à 2, choisi parmi les colorants naturels et les colorants directs non naturels, les précurseurs de colorant d'oxydation, et leurs combinaisons.

Par « colorants naturels », on entend tout colorant ou précurseur de colorant ayant une occurrence naturelle et produit soit par extraction, et éventuellement purification, depuis une matrice végétale, soit par synthèse chimique.

Les colorants naturels additionnels convenables en particulier à la mise en œuvre de l'invention peuvent être choisis par exemple parmi l'acide carminique, l'acide kermésique, l'isatine, les chlorophyllines, l'hématéine, l'hématoxyline, la braziline, la braziléine, la bétanine, les flavonoïdes, les anthocyanes.

On peut également utiliser les extraits ou décoctions contenant ces colorants naturels, et notamment les extraits à base de henné.

La composition peut également comprendre un ou plusieurs colorants directs non naturels additionnels, différents des colorants à caractère hydrophobe décrits plus haut, et choisis parmi des espèces ioniques ou non ioniques, de préférence cationiques ou non ioniques.

A titre d'exemples de colorants directs additionnel convenables, on peut citer les colorants directs azoïques, méthiniques, carbonyles, aziniques, nitrés (hétéro)aryle, tri-(hétéro)aryle méthanes, les porphyrines et les phtalocyanines, seuls ou en mélange.

Plus particulièrement, les colorants azoïques comprennent une fonction -N=N-, dont les deux atomes d'azote ne sont pas simultanément engagés dans un cycle. Il n'est toutefois pas exclu que l'un des deux atomes d'azote de l'enchaînement -N=N- soit engagé dans un cycle.

Les colorants de la famille des méthines sont plus particulièrement des composés comprenant au moins un enchaînement choisi parmi >C=C< et -N=C<, dont les deux atomes ne sont pas simultanément engagés dans un cycle. Il est toutefois précisé que l'un des atomes d'azote ou de carbone des enchaînements peut être engagé dans un cycle. Plus particulièrement, les colorants de cette famille sont issus de composés de type méthine vraie, comprenant un ou plusieurs enchaînements -C=C- précités, azométhine, comprenant au moins un ou plusieurs enchaînements -C=N- avec par exemple les azacarbocyanines et leurs isomères, les diazacarbocyanines et leurs isomères, les tétraazacarbocyanines, les mono- et di- arylméthanes, les indoamines ou diphénylamines, les indophénols, les indoanilines.

Concernant les colorants de la famille des carbonyles, on peut citer par exemple les colorants non naturels choisis parmi les acridone, benzoquinone, anthraquinone, naphtoquinone, benzanthrone, anthranthrone, pyranthrone, pyrazolanthrone, pyrimidinoanthrone, flavanthrone, idanthrone, flavone, (iso)violanthrone, isoindolinone, benzimidazolone, isoquinolinone, anthrapyridone, pyrazoloquinazolone, périnone, quinacridone, quinophthalone, naphtalimide, anthrapyrimidine, dicétopyrrolopyrrole, coumarine.

Concernant les colorants de la famille des azines cycliques, on peut citer notamment les azine, xanthène, thioxanthène, fluorindine, acridine, (di)oxazine, (di)thiazine, pyronine.

Les colorants nitrés (hétéro)aromatiques sont plus particulièrement des colorants directs nitrés benzéniques ou nitrés pyridiniques.

Concernant les colorants de type porphyrines ou phtalocyanines, on peut mettre en œuvre des composés cationiques ou non, comprenant éventuellement un ou plusieurs métaux ou ions métalliques, comme par exemple des métaux alcalins et alcalino-terreux, le zinc et le silicium.

A titre d'exemple de colorants directs de synthèse additionnels particulièrement convenables, on peut citer les colorants nitrés de la série benzénique, les colorants directs azoïques, azométhiniques, méthiniques, les azacarbocyanines comme les tétraazacarbocyanines et les tétraazapentaméthines, les colorants directs quinoniques et en particulier anthraquinoniques, naphtoquinoniques ou benzoquinoniques, les colorants directs aziniques, xanthéniques, triarylméthaniques, indoaminiques, les phtalocyanines et les porphyrines, seuls ou en mélange. De manière encore plus préférée, ces colorants directs additionnels sont choisis parmi les colorants nitrés de la série benzénique, les colorants directs azoïques, azométhiniques, méthiniques, les tétraazacarbocyanines et les tétraazapentaméthines, seuls ou en mélange.

Ces colorants peuvent être des colorants monochromophoriques, c'est-à-dire ne comprenant qu'un seul colorant, ou polychromophoriques, de préférence di- ou tri-chromophoriques, les chromophores pouvant être identiques ou non, de la même famille chimique ou non. A noter qu'un colorant polychromophorique comprend plusieurs radicaux issus chacun d'une molécule absorbant dans le domaine visible, entre 400 et 800 nm. De plus, cette absorbance du colorant ne nécessite ni oxydation préalable de celui-ci, ni association avec d'autre(s) espèce(s) chimique(s).

Dans le cas de colorants polychromophoriques, les chromophores sont reliés entre eux au moyen d'au moins un bras de liaison qui peut être cationique ou non.

Parmi les colorants polychromophoriques, on peut citer plus particulièrement les colorants di- ou tri-chromophoriques azoïques et/ou azométhiniques, tels qu'hydrazoniques, symétriques ou non, comprenant, d'une part, au moins

un hétérocycle aromatique comprenant 5 ou 6 chaînons, éventuellement condensé, comprenant au moins un atome d'azote quaternisé engagé dans ledit hétérocycle et éventuellement au moins un autre hétéroatome, tel que l'azote, le soufre, l'oxygène, et d'autre part, au moins un groupement phényle ou naphtyle éventuellement substitué, éventuellement porteur d'au moins un groupement -OR, avec R représentant un atome d'hydrogène, un radical alkyle éventuellement substitué en $C_1$-$C_6$, un noyau phényle éventuellement substitué, ou au moins un groupement $N(R')_2$, avec R' identiques ou non, représentant un atome d'hydrogène, un radical alkyle éventuellement substitué en $C_1$-$C_6$, un noyau phényle éventuellement substitué, les radicaux R' pouvant former, avec l'atome d'azote auquel ils sont liés, un hétérocycle saturé à 5 ou 6 chaînons, ou bien encore l'un et/ou les deux radicaux R' peuvent former chacun avec l'atome de carbone du cycle aromatique placé en ortho de l'atome d'azote, un hétérocycle saturé à 5 ou 6 chaînons.

A titre d'hétérocycle cationique aromatique, on peut citer, de préférence, les cycles à 5 ou 6 chaînons comprenant 1 à 3 atomes d'azote, de préférence 1 ou 2 atomes d'azote, l'un étant quaternisé, ledit hétérocycle étant par ailleurs éventuellement condensé à un noyau benzénique. Il est à noter de même que l'hétérocycle peut éventuellement comprendre un autre hétéroatome différent de l'azote, comme le soufre ou l'oxygène.

Si les hétérocycles ou groupements phényle ou napthyle sont substitués, ils le sont par exemple par un ou plusieurs radicaux alkyle en $C_1$-$C_8$ éventuellement substitués par un groupement hydroxy, alcoxy en $C_1$-$C_2$, hydroxyalcoxy en $C_2$-$C_4$, acétylamino, amino substitué par un ou deux radicaux alkyle en $C_1$-$C_4$, éventuellement porteurs d'un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote, un atome d'halogène, un groupement hydroxyle, un radical alcoxy en $C_1$-$C_2$, un radical hydroxyalcoxy en $C_2$-$C_4$, un radical amino, un radical amino substitué par un ou deux radicaux alkyle, identiques ou différents, en $C_1$-$C_4$ éventuellement porteurs d'un groupement hydroxyle.

De préférence, le bras de liaison est une chaîne alkyle en $C_1$-$C_{20}$, linéaire, ramifiée ou cyclique, éventuellement interrompue par au moins un hétéroatome, tel que l'azote, l'oxygène et/ou par au moins un groupe comprenant CO, $SO_2$, éventuellement interrompue par au moins un groupement phényle ou naphtyle substitué ou non, éventuellement interrompue par au moins un hétérocycle saturé, insaturé ou aromatique, condensé ou non avec un noyau phényle, ledit hétérocycle comprenant au moins un atome d'azote quaternisé engagé dans ledit cycle et éventuellement au moins un autre hétéroatome, tel que l'oxygène, l'azote ou le soufre, éventuellement interrompue par au moins un groupement ammonium quaternaire substitué par deux groupements alkyle en $C_1$-$C_{15}$, le bras de liaison ne comprenant pas de groupement nitro, nitroso ou peroxo.

Si les hétérocycles ou noyaux aromatiques sont substitués, ils le sont par exemple par un ou plusieurs radicaux alkyle en $C_1$-$C_8$ éventuellement substitués par un groupement hydroxy, alcoxy en $C_1$-$C_2$, hydroxyalcoxy en $C_2$-$C_4$, acétylamino, amino substitué par un ou deux radicaux alkyle, identiques ou différents, en $C_1$-$C_4$, éventuellement porteurs d'un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote, un atome d'halogène, un groupement hydroxyle, un radical alcoxy en $C_1$-$C_2$, un radical hydroxyalcoxy en $C_2$-$C_4$, un radical amino, un radical amino substitué par un ou deux radicaux alkyle, identiques ou différents, en $C_1$-$C_4$ éventuellement porteurs d'un groupement hydroxyle.

La liaison entre le bras de liaison et chaque chromophore se fait en général au moyen d'un hétéroatome substituant le noyau phényle ou napthyle, ou au moyen de l'atome d'azote quaternisé de l'hétérocycle cationique.

Parmi les colorants directs monochromophoriques azoïques, azométhines, méthines ou tétraazapentaméthines utilisables selon l'invention, on peut citer les colorants cationiques décrits dans les demandes de brevets WO 95/15144, WO 95/01772 et EP 714954, FR 2189006, FR 2285851, FR-2140205, EP 1378544, EP 1674073.

Ainsi, on peut tout notamment citer les colorants directs cationiques correspondants aux formules suivantes :

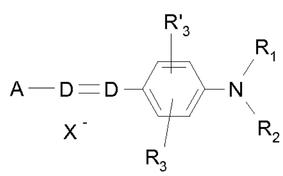

dans laquelle :

D représente un atome d'azote ou le groupement -CH,

$R_1$ et $R_2$, identiques ou différents, représentent un atome d'hydrogène, un radical alkyle en $C_1$-$C_4$ pouvant être substitué par un radical -CN, -OH ou $-NH_2$ ou forment, avec un atome de carbone du cycle benzénique, un hétérocycle éventuellement oxygéné ou azoté, pouvant être substitué par un ou plusieurs radicaux alkyle en $C_1$-$C_4$, un radical 4'-aminophényle,

$R_3$ et $R'_3$, identiques ou différents, représentent un atome d'hydrogène ou d'halogène choisi parmi le chlore, le brome, l'iode et le fluor, un radical cyano, alkyl en $C_1$-$C_4$, alcoxy en $C_1$-$C_4$ ou acétyloxy,
$X^-$ représente un anion, de préférence choisi parmi le chlorure, le méthyl sulfate et l'acétate,
A représente un groupement choisi parmi les structures suivantes :

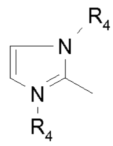 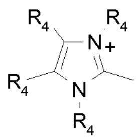 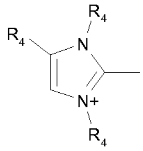 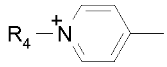 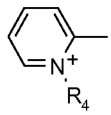

dans lesquelles $R_4$ représente un radical alkyle en $C_1$-$C_4$ pouvant être substitué par un radical hydroxyle,

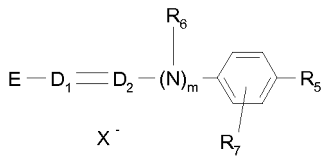

dans lesquelles :

$R_5$ représente un atome d'hydrogène, un radical alcoxy en $C_1$-$C_4$, un atome d'halogène tel que le brome, le chlore, l'iode ou le fluor,
$R_6$ représente un atome d'hydrogène, un radical alkyle en $C_1$-$C_4$ ou forme, avec un atome de carbone du cycle benzénique, un hétérocycle éventuellement oxygéné et/ou substitué par un ou plusieurs groupements alkyle en $C_1$-$C_4$,
$R_7$ représente un atome d'hydrogène ou d'halogène tel que le brome, le chlore, l'iode ou le fluor,
$D_1$ et $D_2$, identiques ou différents, représentent un atome d'azote ou le groupement -CH,
m = 0 ou 1,
$X^-$ représente un anion cosmétiquement acceptable, et de préférence choisi parmi le chlorure, le méthyl sulfate et l'acétate,
E représente un groupement choisi par les structures suivantes :

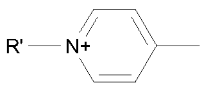 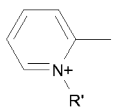 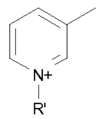

dans lesquelles R' représente un radical alkyle en $C_1$-$C_4$.

Lorsque m = 0 et que $D_1$ représente un atome d'azote, alors E peut également désigner un groupement de structure suivante :

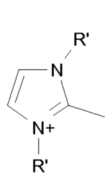

dans laquelle R' représente un radical alkyle en $C_1$-$C_4$.
Parmi les composés précités, on utilise tout particulièrement les composés suivants :

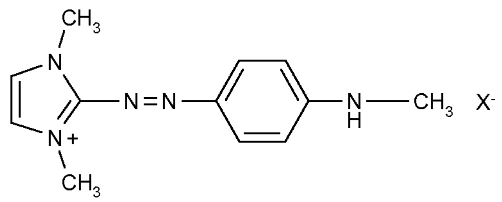

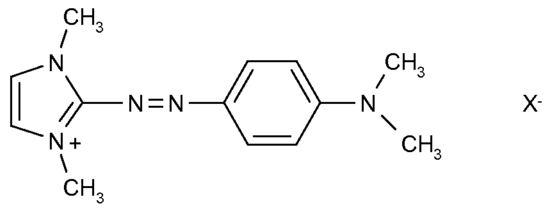

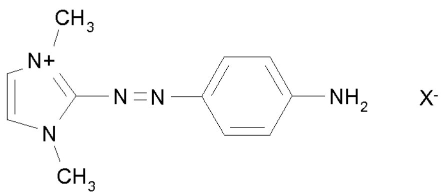

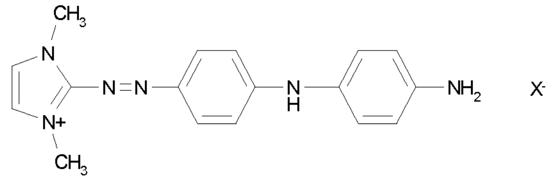

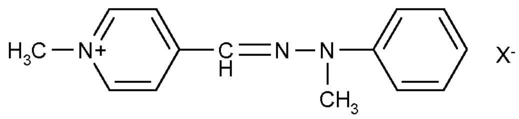

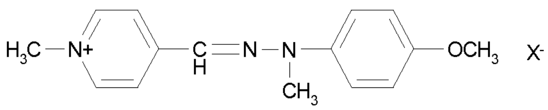

Parmi les colorants de type tétraazapentaméthiniques utilisables selon l'invention, on peut citer les composés suivants figurant dans le tableau ci-dessous :

| | |
|---|---|
| 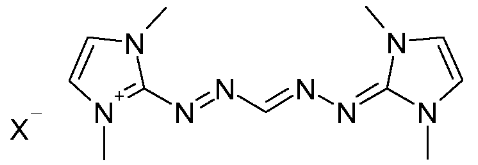 | 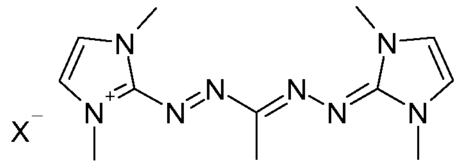 |
| 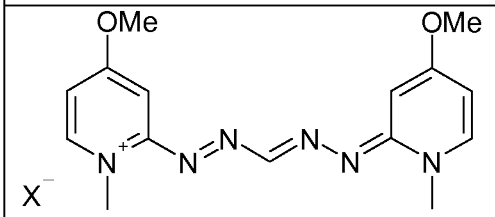 | 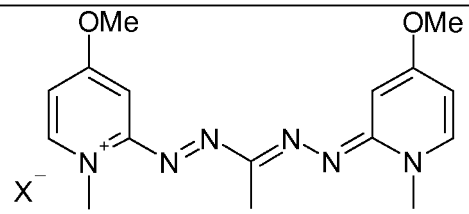 |
| 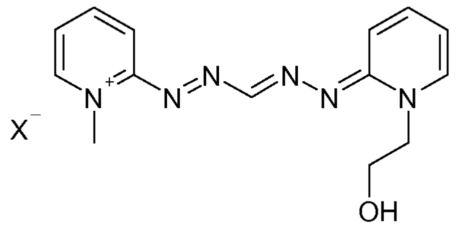 | 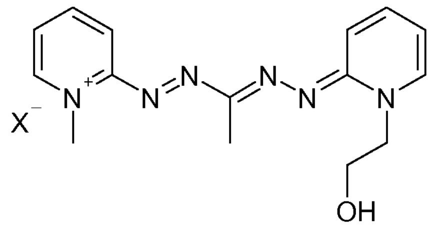 |
| 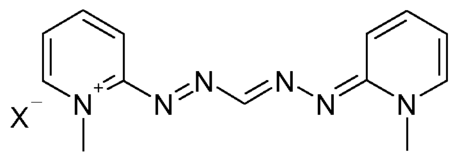 | 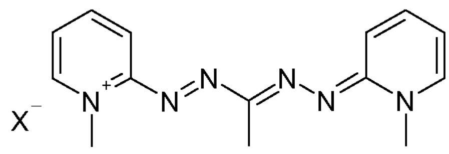 |
| 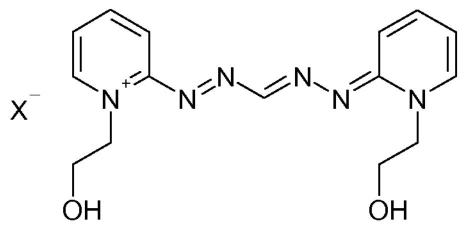 | 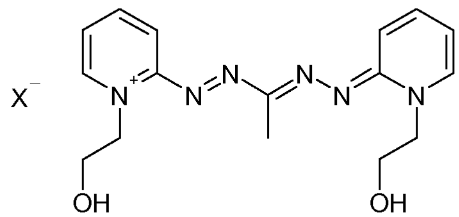 |

$X^-$ représente un anion, de préférence choisi parmi le chlorure, l'iodure, le méthylsulfate, l'éthylsulfate, l'acétate.

La composition tinctoriale peut également comprendre un ou plusieurs précurseurs de colorants d'oxydation, plus particulièrement une ou plusieurs bases d'oxydation, éventuellement combinée(s) à un ou plusieurs coupleurs.

A titre d'exemple, les bases d'oxydation sont choisies parmi les paraphénylènediamines, les bis-phénylalkylènediamines, les para-aminophénols, les ortho-aminophénols, les bases hétérocycliques, et leurs sels d'addition.

Parmi les paraphénylènediamines, on peut citer, à titre d'exemple, la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino N,N-diéthyl-3-méthylaniline, la N,N-bis-(β-hydroxyéthyl)-paraphénylènediamine, la 4-N,N-bis-(β-hydroxyéthyl)amino-2-méthylaniline, la 4-N,N-bis-(β-hydroxyéthyl)amino-2-chloroaniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylène-diamine, la 4-aminophénylpyrrolidine, la 2-thiényl-paraphénylènediamine, le 2-β-hydroxyéthylamino-5-aminotoluène, la 3-hydroxy-1-(4'-aminophényl)pyrrolidine, et leurs sels d'addition avec un acide.

Parmi les paraphénylènediamines citées ci-dessus, la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylènediamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, la 2-β-acétylaminoéthyloxy-paraphénylènediamine, et leurs sels d'addition avec un acide, sont particulièrement préférées.

Parmi les bis-phénylalkylènediamines, on peut citer, à titre d'exemple, le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino,3'-méthylphényl)-éthylènediamine, le 1,8-bis-(2,5-diamino-phénoxy)-3,6-dioxaoctane, et leurs sels d'addition.

Parmi les para-aminophénols, on peut citer, à titre d'exemple, le para-aminophénol, le 4-amino-3-méthylphénol, le 4-amino-3-fluorophénol, le 4-amino-3-chlorophénol, le 4-amino-3-hydroxyméthylphénol, le 4-amino-2-méthylphénol, le 4-amino-2-hydroxyméthylphénol, le 4-amino-2-méthoxyméthylphénol, le 4-amino-2-aminométhylphénol, le 4-amino-2-(β-hydroxyéthyl-aminométhyl)-phénol, le 4-amino-2-fluorophénol, et leurs sels d'addition avec un acide.

Parmi les ortho-aminophénols, on peut citer, à titre d'exemple, le 2-aminophénol, le 2-amino-5-méthylphénol, le 2-amino-6-méthylphénol, le 5-acétamido-2-aminophénol, et leurs sels d'addition.

Parmi les bases hétérocycliques, on peut citer, à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diaminopyridine, la 2-(4-méthoxyphényl)amino-3-aminopyridine, la 3,4-diaminopyridine, et leurs sels d'addition.

D'autres bases d'oxydation pyridiniques utiles dans la présente invention sont les bases d'oxydation 3-amino-pyrazolo-[1,5-a]-pyridines et leurs sels d'addition décrits, par exemple, dans la demande de brevet FR 2801308. A titre d'exemple, on peut citer la pyrazolo[1,5-a]pyridin-3-ylamine, la 2-acétylamino pyrazolo-[1,5-a] pyridin-3-ylamine, la 2-morpholin-4-yl-pyrazolo[1,5-a]pyridin-3-ylamine, l'acide 3-amino-pyrazolo[1,5-a]pyridin-2-carboxylique, la 2-méthoxy-pyrazolo[1,5-a]pyridine-3-ylamino, le (3-amino-pyrazolo[1,5-a]pyridine-7-yl)-méthanol, le 2-(3-amino-pyrazolo[1,5-a]pyridine-5-yl)-éthanol, le 2-(3-amino-pyrazolo[1,5-a]pyridine-7-yl)-éthanol, le (3-amino-pyrazolo[1,5-a]pyridine-2-yl)-méthanol, la 3,6-diamino-pyrazolo[1,5-a]pyridine, la 3,4-diamino-pyrazolo[1,5-a]pyridine, la pyrazolo[1,5-a]pyridine-3,7-diamine, la 7-morpholin-4-yl-pyrazolo[1,5-a]pyridin-3-ylamine, la pyrazolo[1,5-a]pyridine-3,5-diamine, la 5-morpholin-4-yl-pyrazolo[1,5-a]pyridin-3-ylamine, le 2-[(3-amino-pyrazolo[1,5-a]pyridin-5-yl)-(2-hydroxyéthyl)-amino]-éthanol, le 2-[(3-amino-pyrazolo[1,5-a]pyridin-7-yl)-(2-hydroxyéthyl)-amino]-éthanol, la 3-amino-pyrazolo[1,5-a]pyridine-5-ol, 3-amino-pyrazolo[1,5-a]pyridine-4-ol, la 3-amino-pyrazolo[1,5-a]pyridine-6-ol, la 3-amino-pyrazolo[1,5-a]pyridine-7-ol, ainsi que leurs sels d'addition.

Parmi les dérivés pyrimidiniques, on peut citer les composés décrits par exemple dans les brevets DE 2359399, JP 88-169571, JP 05-63124, EP 0770375 et la demande de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et les demandes de brevet WO 94/08969, WO 94/08970, FR-A-2 733 749 et DE 195 43 988, comme le 4,5-diamino-1-méthylpyrazole, le 4,5-diamino-1-(β-hydroxyéthyl)pyrazole, le 3,4-diaminopyrazole, le 4,5-diamino 1-(4'-chlorobenzyl)pyrazole, le 4,5-diamino-1,3-diméthylpyrazole, le 4,5-diamino-3-méthyl-1-phénylpyrazole, le 4,5-diamino-1-méthyl-3-phénylpyrazole, le 4-amino-1,3-diméthyl-5-hydrazinopyrazole, le 1-benzyl-4,5-diamino-3-méthylpyrazole, le 4,5-diamino-3-tert-butyl-1-méthylpyrazole, le 4,5-diamino-1-tert-butyl-3-méthylpyrazole, le 4,5-diamino-1-(β-hydroxyéthyl)-3-méthylpyrazole, le 4,5-diamino-1-éthyl 3-méthylpyrazole, le 4,5-diamino-1-éthyl-3-(4'-méthoxyphényl)pyrazole, le 4,5-diamino-1-éthyl-3-hydroxyméthylpyrazole, le 4,5-diamino-3-hydroxyméthyl-1-méthylpyrazole, le 4,5-diamino-3-hydroxyméthyl-1-isopropylpyrazole, le 4,5-diamino-3-méthyl-1-isopropylpyrazole, le 4-amino-5-(2'-aminoéthyl)amino-1,3-diméthylpyrazole, le 3,4,5-triaminopyrazole, le 1-méthyl-3,4,5-triaminopyrazole, le 3,5-diamino-1-méthyl-4-méthylaminopyrazole, le 3,5-diamino-4-(β-hydroxyéthyl)amino-1-méthylpyrazole, et leurs sels d'addition. On peut aussi utiliser le 4-5-diamino-1-(β-méthoxyéthyl)pyrazole.

A titre de dérivés pyrazoliques, on peut également citer les diamino-N,N-dihydropyrazolopyrazolones et notamment celles décrites dans la demande FR 2886136, telles que les composés suivants et leurs sels d'addition : 2,3-diamino-6,7-dihydro-1H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diethyl-5-(pyrrolidin-1-yl)-1,2-dihydro-pyrazol-3-one, 4-amino-5-(3-dimethylamino-pyrrolidin-1-yl)-1,2-diethyl-1,2-dihydro-pyrazol-3-one, 2,3-diamino-6-hydroxy-6,7-dihydro-1H,5H-pyrazolo[1,2-a]pyrazol-1-one.

A titre de bases hétérocycliques, on utilisera préférentiellement le 4,5-diamino-1-(β-hydroxyéthyl)pyrazole et/ou

la 2,3-diamino-6,7-dihydro-1H,5H-pyrazolo[1,2,A]pyrazol-1-one, et leurs sels d'addition.

La composition selon l'invention peut éventuellement comprendre un ou plusieurs coupleurs choisis avantageusement parmi ceux conventionnellement utilisés pour la teinture des fibres kératiniques.

Parmi ces coupleurs, on peut notamment citer les méta-phénylènediamines, les méta-aminophénols, les méta-diphénols, les coupleurs naphtaléniques, les coupleurs hétérocycliques, ainsi que leurs sels d'addition.

A titre d'exemple, on peut citer le 1,3-dihydroxybenzène, le 1,3-dihydroxy-2-méthylbenzène, le 4-chloro-1,3-dihydroxybenzène, le 2,4-diamino-1-(β-hydroxyéthyloxy)benzène, le 2-amino-4-(β-hydroxyéthylamino)-1-méthoxybenzène, le 1,3-diaminobenzène, le 1,3-bis-(2,4-diaminophénoxy)propane, la 3-uréidoaniline, le 3-uréido-1-diméthylaminobenzène, le sésamol, le 1-β-hydroxyéthylamino-3,4-méthylènedioxybenzène, l'a-naphtol, le 2-méthyl-1-naphtol, le 6-hydroxyindole, le 4-hydroxyindole, le 4-hydroxy-N-méthylindole, la 2-amino-3-hydroxypyridine, la 6-hydroxybenzomorpholine, la 3,5-diamino-2,6-diméthoxypyridine, le 1-N-(β-hydroxyéthyl)amino-3,4-méthylènedioxybenzène, le 2,6-bis-(β-hydroxyéthylamino)toluène, la 6-hydroxyindoline, la 2,6-dihydroxy-4-méthylpyridine, la 1-H-3-méthylpyrazole-5-one, la 1-phényl-3-méthylpyrazole-5-one, le 2,6-diméthylpyrazolo-[1,5-b]-1,2,4-triazole, le 2,6-diméthyl-[3,2-c]-1,2,4-triazole, le 6-méthylpyrazolo-[1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation, quand elles sont présentes dans la composition, représentent avantageusement de 0,0001 à 10 % en poids par rapport au poids de la composition, et de préférence de 0,005 à 5 % en poids par rapport au poids de la composition.

Le ou les coupleurs, s'ils sont présents, représentent avantageusement de 0,0001 à 10 % en poids par rapport au poids de la composition, et de préférence de 0,005 à 5 % en poids par rapport au poids de la composition.

Lorsqu'ils sont présents, le ou les colorants additionnels représentent de 0,01 à 10 % en poids et de préférence de 0,5 à 5 % en poids par rapport au poids de la composition.

La composition tinctoriale selon l'invention peut également comprendre un ou plusieurs agents de conditionnement.

A titre d'exemple, on peut citer les silicones linéaires, cycliques, ramifiées ou non ramifiées, volatiles ou non volatiles. Ces silicones peuvent se présenter sous forme d'huiles, de résines ou de gommes, elles peuvent en particulier être des polyorganosiloxanes insolubles dans le milieu cosmétiquement acceptable.

Les organopolysiloxanes sont définis plus en détail dans l'ouvrage de Walter NOLL "Chemistry and Technology of Silicones" (1968) Academic Press. Ils peuvent être volatils ou non volatils.

Lorsqu'elles sont volatiles, les silicones sont plus particulièrement choisies parmi celles possédant un point d'ébullition compris entre 60° C et 260° C.

A titre d'agent de conditionnement, on peut aussi utiliser les polymères cationiques, tels que les polyquaterniums 22, 6, 10, 11, 35 et 37 et le chlorure d'hexadiméthrine.

La concentration en agent(s) de conditionnement dans la ou les compositions utiles dans l'invention peut varier de 0,01 à 10 % en poids par rapport au poids total de la composition, de préférence de 0,05 à 5 % et plus préférentiellement encore de 0,1 à 3 %.

La composition selon l'invention peut également comprendre un ou plusieurs agents épaississants organiques.

Les agents épaississants organiques peuvent être choisis parmi les amides d'acides gras, tels que les diéthanol- ou monoéthanol-amide de coprah et le monoéthanolamide d'acide alkyl éther carboxylique oxyéthyléné, les épaississants polymériques tels que les épaississants cellulosiques, en particulier l'hydroxyéthylcellulose, l'hydroxypropylcellulose et la carboxyméthylcellulose, la gomme de guar et ses dérivés tels que l'hydroxypropylguar, les gommes d'origine microbienne telles que la gomme de xanthane et la gomme de scléroglucane, les homopolymères réticulés d'acide acrylique ou d'acide acrylamidopropanesulfonique et les polymères associatifs, c'est-à-dire des polymères comprenant des zones hydrophiles, et des zones hydrophobes à chaîne grasse capables, dans un milieu aqueux, de s'associer réversiblement entre eux ou avec d'autres molécules.

Selon un mode de réalisation particulier, l'épaississant est polymérique et choisi parmi les épaississants cellulosiques tels que l'hydroxyéthylcellulose, l'hydroxypropylcellulose et la carboxyméthylcellulose, la gomme de guar et ses dérivés telles que l'hydroxypropylguar, les gommes d'origine microbienne telles que la gomme de xanthane et la gomme de scléroglucane, les homopolymères réticulés d'acide acrylique ou d'acide acrylamidopropanesulfonique.

En ce qui concerne les agents épaississants associatifs, on peut mettre en œuvre un ou plusieurs polymères de nature non ionique ou ionique, de préférence anionique ou cationique.

Leur structure chimique comprend plus particulièrement au moins une zone hydrophile et au moins une zone hydrophobe. Par « groupement hydrophobe », on entend un radical ou polymère à chaîne hydrocarbonée, saturée ou non, linéaire ou ramifiée, comprenant au moins 8 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel. A titre d'exemple, le groupement hydrophobe peut être issu d'un alcool gras, tel que l'alcool stéarylique, l'alcool dodécylique, l'alcool décylique. Il peut également désigner un polymère hydrocarboné, tel que par exemple le polybutadiène.

Parmi les polymères amphiphiles anioniques comportant au moins une chaîne grasse ou hydrophobe, on peut citer :

- (I) les polymères comportant au moins un motif hydrophile, et au moins un motif éther d'allyle à chaîne grasse, plus particulièrement ceux dont le motif hydrophile est constitué par un monomère anionique insaturé éthylénique, avantageusement par un acide carboxylique vinylique et tout particulièrement par un acide acrylique ou un acide méthacrylique, et leurs mélanges, et dont le motif éther d'allyle à chaîne grasse correspond au monomère de formule (A) suivante :

$$CH_2{=}CR'CH_2OB_nR \qquad (A)$$

dans laquelle R' désigne H ou $CH_3$, B désigne le radical éthylèneoxy, n est nul ou désigne un entier allant de 1 à 100, R désigne un radical hydrocarboné choisi parmi les radicaux alkyle, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (A) plus particulièrement préféré est un motif dans lequel R' désigne H, n est égal à 10, et R désigne un radical stéaryl en $C_{18}$.

Parmi ces polymères anioniques à chaîne grasse, on préfère les polymères formés à partir de 20 à 60 % en poids d'acide acrylique et/ou d'acide méthacrylique, de 5 à 60 % en poids de (méth)acrylates d'alkyles inférieurs, de 2 à 50 % en poids d'éther d'allyl à chaîne grasse de formule (A), et de 0 à 1 % en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, de polyéthylèneglycol (10 OE) éther d'alcool stéarylique, tels que le Steareth 10, notamment ceux vendus par la société ALLIED COLLOIDS sous les dénominations Salcare SC 80 et Salcare SC90, qui sont des émulsions aqueuses à 30 % d'un terpolymère réticulé d'acide méthacrylique, d'acrylate d'éthyle et de steareth-10-allyl éther (40/50/10).

- (II) les polymères comportant au moins un motif hydrophile de type acide carboxylique insaturé oléfinique, et au moins un motif hydrophobe de type ester d'alkyl en $C_{10}$-$C_{30}$ d'acide carboxylique insaturé.

De préférence, ces polymères sont choisis parmi ceux dont le motif hydrophile de type acide carboxylique insaturé oléfinique correspond au monomère de formule (B) suivante :

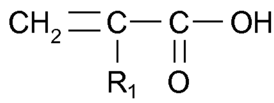

(B)

dans laquelle, $R_1$ désigne H ou $CH_3$ ou $C_2H_5$, c'est-à-dire des motifs acide acrylique, acide méthacrylique ou acide éthacrylique, et dont le motif hydrophobe de type ester d'alkyl en $C_{10}$-$C_{30}$ d'acide carboxylique insaturé correspond au monomère de formule (C) suivante :

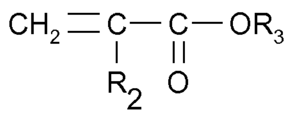

(C)

dans laquelle, $R_2$ désigne H ou $CH_3$ ou $C_2H_5$, c'est-à-dire des motifs acrylates, méthacrylates ou éthacrylates, et de préférence H c'est-à-dire des motifs acrylates ou $CH_3$ c'est-à-dire des motifs méthacrylates, $R_3$ désignant un radical alkyle en $C_{10}$-$C_{30}$, et de préférence en $C_{12}$-$C_{22}$.

Les esters d'alkyles en $C_{10}$-$C_{30}$ d'acides carboxyliques insaturés sont, par exemple, l'acrylate de lauryle, l'acrylate de stéaryle, l'acrylate de décyle, l'acrylate d'isodécyle, l'acrylate de dodécyle, et les méthacrylates correspondants,tels que le méthacrylate de lauryle, le méthacrylate de stéaryle, le méthacrylate de décyle, le méthacrylate d'isodécyle et le méthacrylate de dodécyle.

Parmi ce type de polymères anioniques à chaîne grasse, on utilisera plus particulièrement des polymères

formés à partir d'un mélange de monomères comprenant :

(i) essentiellement de l'acide acrylique,
(ii) un ester de formule (C) décrite ci-dessus et dans laquelle $R_2$ désigne H ou $CH_3$, $R_3$ désignant un radical alkyle ayant de 12 à 22 atomes de carbone,
(iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de polymères anioniques à chaîne grasse, on utilisera plus particulièrement ceux constitués de 95 à 60 % en poids d'acide acrylique, qui correspond au motif hydrophile, de 4 à 40 % en poids d'acrylate d'alkyles en $C_{10}$-$C_{30}$, qui correspond au motif hydrophobe, et de 0 à 6 % en poids de monomère polymérisable réticulant, ou bien ceux constitués de 98 à 96 % en poids d'acide acrylique, qui correspond au motif hydrophile, de 1 à 4 % en poids d'acrylate d'alkyles en $C_{10}$-$C_{30}$, qui correspond au motif hydrophobe, et de 0,1 à 0,6 % en poids de monomère polymérisable réticulant, tel que ceux décrits précédemment.
Parmi lesdits polymères ci-dessus, on préfère tout particulièrement selon la présente invention, les produits vendus par la société GOODRICH sous les dénominations commerciales Pemulen TR1, Pemulen TR2, Carbopol 1382, et encore plus préférentiellement le Pemulen TR1, et le produit vendu par la société S.E.P.P.I.C. sous la dénomination Coatex SX .

- (III) les terpolymères d'anhydride maléique/α-oléfine en $C_{30}$-$C_{38}$/maléate d'alkyle, tel que le produit copolymère anhydride maléique/α-oléfine en $C_{30}$-$C_{38}$/maléate d'isopropyle vendu sous le nom Performa V 1608 par la société NEWPHASE TECHNOLOGIES.
- (IV) les terpolymères acryliques comprenant :

  (a) de 20% à 70% en poids d'un acide carboxylique à insaturation-α, β-monoéthylénique,
  (b) de 20 à 80% en poids d'un monomère à insaturation-α,β-monoéthylénique non tensioactif différent de (a),
  (c) de 0,5 à 60% en poids d'un monouréthane non ionique, qui est le produit de réaction d'un tensioactif monohydrique avec un monoisocyanate à insaturation monoéthylénique, tels que ceux décrits dans la demande de brevet EP-A-0173109 et plus particulièrement un terpolymère acide méthacrylique/acrylate de méthyle/diméthyl métaisopropényl benzyl isocyanate d'alcool béhényle éthoxylé (400E) en dispersion aqueuse à 25%.

- (V) les copolymères comportant parmi leurs monomères un acide carboxylique à insaturation-α,β-monoéthylénique et un ester d'acide carboxylique à insaturation-α,β-monoéthylénique et d'un alcool gras ($C_8$-$C_{30}$)oxyalkyléné.

Préférentiellement, ces composés comprennent également, comme monomère, un ester d'acide carboxylique à insaturation-α,β-monoéthylénique et d'alcool en $C_1$-$C_4$.
A titre d'exemple de ce type de composé, on peut citer l'Aculyn 22 vendu par la société ROHM & HAAS, qui est un terpolymère acide méthacrylique/acrylate d'éthyle/méthacrylate de stéaryle oxyalkyléné.
Les polymères amphiphiles non ioniques à chaîne grasse ou hydrophobe sont choisis de préférence parmi :

- (1) les celluloses modifiées par des groupements comportant au moins une chaîne grasse, comme notamment :

  - les hydroxyéthylcelluloses modifiées par des groupements comportant au moins une chaîne grasse, tels que des groupes alkyle, arylalkyle, alkylaryle, et leurs mélanges, et dans lesquels les groupes alkyle sont de préférence en $C_8$-$C_{22}$, comme le produit Natrosol Plus Grade 330 CS, comprenant des alkyles en $C_{16}$, vendu par la société AQUALON, ou le produit Bremocoll Ehm 100 vendu par la société BEROL NOBEL,
  - celles modifiées par des groupes polyalkylène glycol éther d'alkyl phénol, tel que le produit Amercell Polymer HM-1500, qui est un polyéthylène glycol (15) éther de nonyl phénol vendu par la société AMERCHOL,

- (2) les hydroxypropylguars modifiés par des groupements comportant au moins une chaîne grasse, tel que le produit Esaflor HM 22, comprenant une chaîne alkyle en $C_{22}$, vendu par la société LAMBERTI, les produits RE210-18, comprenant une chaîne alkyle en $C_{14}$, et RE205-1 comprenant une chaîne alkyle en $C_{20}$, vendus par la société RHONE POULENC,
- (3) les copolymères de vinyl pyrrolidone et de monomères hydrophobes à chaîne grasse, comme par exemple :

  - les produits Aataron V216 et Ganex V216, qui sont des copolymères vinylpyrrolidone/hexadécène, vendus par la société I.S.P.,

- les produits Antaron V220 et Ganex V220, qui sont des copolymères vinylpyrrolidone/eicosène, vendu par la société I.S.P.,

- (4) les copolymères de méthacrylates ou d'acrylates d'alkyles en $C_1$-$C_6$ et de monomères amphiphiles comportant au moins une chaîne grasse, tels que par exemple le copolymère acrylate de méthyle/acrylate de stéaryle oxyéthyléné vendu par la société GOLDSCHMIDT sous la dénomination Antil 208,
- (5) les copolymères de méthacrylates ou d'acrylates hydrophiles et de monomères hydrophobes comportant au moins une chaîne grasse, tels que par exemple le copolymère méthacrylate de polyéthylèneglycol/méthacrylate de lauryle,
- (6) les polymères à squelette aminoplaste éther possédant au moins une chaîne grasse, tels que les composés Pure Thix proposés par la société SUD-CHEMIE,
- (7) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, les polyéthers polyuréthanes comportent au moins deux chaînes grasses hydrocarbonées, ayant de 8 à 30 atomes de carbone, séparées par une séquence hydrophile, les chaînes hydrocarbonées pouvant être des chaînes pendantes ou des chaînes en bout de séquence hydrophile.

A titre d'exemples de polyéthers polyuréthanes non ioniques à chaîne grasse utilisables dans l'invention, on peut utiliser le Rhéolate 205 à fonction urée vendu par la société RHEOX ou encore les Rhéolates 208, 204 et 212, ainsi que l'Acrysol RM 184, l'Aculyn, Acrysol 44 et 46 de la société ROHM & HAAS, l'Aculyn 46 étant un polycondensat de polyéthylèneglycol à 150 ou 180 moles d'oxyde d'éthylène, d'alcool stéarylique et de méthylène bis(4-cyclohexylisocyanate) (SMDI), à 15 % en poids dans une matrice de maltodextrine (4 %) et d'eau (81 %), l'Aculyn 44 étant un polycondensat de polyéthylèneglycol à 150 ou 180 moles d'oxyde d'éthylène, d'alcool décylique et de méthylène bis(4-cyclohexylisocyanate) (SMDI), à 35 % en poids dans un mélange de propylèneglycol (39 %) et d'eau (26 %).

On peut également citer le produit Elfacos T210 à chaîne alkyle en $C_{12}$-$C_{14}$ et le produit Elfacos T212 à chaîne alkyle en $C_{18}$ de chez AKZO, ainsi que le produit DW 1206B de chez ROHM & HAAS à chaîne alkyle en $C_{20}$ et à liaison uréthanne, proposé à 20 % en matière sèche dans l'eau.

On peut aussi utiliser des solutions ou dispersions de ces polymères, notamment dans l'eau ou en milieu hydroalcoolique. A titre d'exemple de tels polymères, on peut citer le Rhéolate 255, le Rhéolate 278 et le Rhéolate 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Les polymères amphiphiles cationiques comportant au moins une chaîne grasse ou hydrophobe utilisés peuvent notamment être choisis parmi les dérivés de cellulose quaternisée, les polyuréthanes cationiques, les polyvinyllactames cationiques, et de préférence parmi les dérivés de cellulose quaternisée.

A titre d'exemple de polymères de ce type, on peut citer en particulier :

- les celluloses quaternisées modifiées par des groupements comportant au moins une chaîne grasse, tels que les groupes alkyle, arylalkyle, alkylaryle comportant au moins 8 atomes de carbone, et leurs mélanges,
- les hydroxyéthylcelluloses quaternisées modifiées par des groupements comportant au moins une chaîne grasse, tels que les groupes alkyle, arylalkyle, alkylaryle comportant au moins 8 atomes de carbone, et leurs mélanges.

Les radicaux alkyles portés par les celluloses ou les hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer, comme exemples d'alkylhydroxyéthylcelluloses quaternisées à chaînes grasses en $C_8$-$C_{30}$, les produits Quatrisoft LM 200, Quatrisoft LM-X 529-18-A, Quatrisoft LM-X 529-18B (alkyle en $C_{12}$) et Quatrisoft LM-X 529-8 (alkyle en $C_{18}$) commercialisés par la société AMERCHOL et les produits Crodacel QM, Crodacel QL (alkyle en $C_{12}$) et Crodacel QS (alkyle en C18) commercialisés par la société CRODA.

La teneur en polymères épaississants, s'ils sont présents, varie habituellement de 0,05% à 5% en poids, par rapport au poids de la composition de coloration.

Selon un mode de réalisation particulièrement avantageux, la composition tinctoriale selon l'invention comprend un ou plusieurs agents tensioactifs. Ces derniers peuvent être indifféremment choisis, seuls ou en mélange, parmi les tensioactifs anioniques, amphotères, non ioniques, zwittérioniques et cationiques.

En ce qui concerne les tensioactifs anioniques, on utilise habituellement les sels, en particulier les sels de métaux alcalins tels que les sels de sodium, les sels d'ammonium, les sels d'amines, les sels d'aminoalcools et les sels

de métaux alcalino-terreux comme le magnésium, et les sels des composés suivants, seuls ou en mélange :

- les alkylsulfates, les alkyléthersulfates, les alkylamidoéthersulfates, les alkylarylpolyéthersulfates,
- les alkylsulfonates, les alkylamidesulfonates, les alkylarylsulfonates,
- les alkylsulfosuccinates, les alkyléthersulfosuccinates, les alkylamide-sulfosuccinates,
- les alkylsulfoacétates,
- les acylsarconisates,
- les acylglutamates,
- les esters d'alkyle et d'acides polyglycoside-carboxyliques tels que les glucoside-citrates d'alkyle, les polyglycoside-tartrates d'alkyle, et les polyglycoside-sulfosuccinates d'alkyle,
- les alkylsulfosuccinamates,
- les acyliséthionates, les N-acyltaurates, les acyllactylates,
- les acides d'alkyl-D-galactoside uroniques,
- les acides alkyléther-carboxyliques polyoxyalkylénés, les acides alkylaryléther-carboxyliques polyoxyalkylénés, les acides alkylamidoéther carboxyliques polyoxyalkylénés,

le groupe alkyle ou acyle (RCO-) de ces composés comportant de 10 à 24 atomes de carbone et le groupe aryle désignant de préférence un groupe phényle ou benzyle, le nombre de groupements oxyalkylénés, et de préférence oxyéthylénés, est compris entre 2 et 50.

Pour ce qui concerne les tensioactifs non ioniques, ces derniers peuvent être avantageusement choisis parmi les composés suivants, seuls ou en mélange :

- les alcools gras polyéthoxylés, polypropoxylés et polyglycérolés,
- les alpha-diols polyéthoxylés, polypropoxylés et polyglycérolés,
  le nombre de groupements oxyde d'éthylène ou oxyde de propylène allant de 2 à 50, le nombre de groupements glycérol allant de 2 à 30,
- les condensats d'oxyde d'éthylène et d'oxyde de propylène sur des alcools gras,
- les amides gras polyéthoxylés ayant de 2 à 30 moles d'oxyde d'éthylène,
- les amides gras polyglycérolés comportant de 1 à 5 groupements glycérol,
- les amines grasses polyéthoxylées ayant de 2 à 30 moles d'oxyde d'éthylène,
- les esters éthoxylés d'acides gras du sorbitane ayant de 2 à 30 moles d'oxyde d'éthylène, les esters d'acides gras du saccharose,
- les alkylpolyglucosides, les dérivés de N-alkylglucamine, ces composés comprenant au moins une chaîne alkyle ou alcényle comprenant 10 à 24 atomes de carbone.
- les copolymères d'oxyde d'éthylène et d'oxyde de propylène.

Les tensioactifs cationiques, entrant dans la composition selon l'invention, peuvent notamment être choisis parmi les composés suivants, seuls ou en mélange :

- les amines grasses primaires, secondaires ou tertiaires éventuellement polyéthoxylées (2 à 30 moles d'oxyde d'éthylène) et leurs sels,
- les sels d'ammonium quaternaire, tels que les chlorures et les bromures de tétraalkylammonium, d'alkylamidoalkyltrialkylammonium, de trialkylbenzylammonium, de trialkylhydroxyalkylammonium, d'alkylpyridinium,
- les dérivés d'alkylimidazoline,

ces composés comprenant au moins une chaîne alkyle comprenant 10 à 24 atomes de carbone.

Enfin, les tensioactifs amphotères peuvent être choisis parmi les composés suivants, seuls ou en mélange :

- les dérivés d'amines aliphatiques secondaires ou tertiaires, dans lesquels le groupe aliphatique est une chaîne linéaire ou ramifiée comportant 10 à 24 atomes de carbone et comprenant au moins un groupe anionique hydrosolubilisant tel que, par exemple, un groupe carboxylate, sulfonate, sulfate, phosphate ou phosphonate,
- les alkylbétaïnes, les sulfobétaïnes, les alkylamidoalkyl($C_6$-$C_8$)bétaïnes, les alkylamidoalkyl($C_6$-$C_8$)sulfobétaïnes,

ces composés comprenant une au moins chaîne alkyle comprenant de 10 à 24 atomes de carbone.

De préférence, les tensioactifs sont non ioniques, anioniques ou amphotères et de manière encore plus préférée, non ioniques.

Habituellement, les agents tensioactifs représentent de 0,01 à 50 % en poids, de préférence de 0,1 à 25 % en poids par rapport au poids de la composition.

La composition tinctoriale selon l'invention peut également comprendre divers adjuvants utilisés classiquement dans les compositions pour la teinture des cheveux, tels que par exemple des polymères cationiques, anioniques, non ioniques, amphotères, zwittérioniques différents des épaississants mentionnés auparavant, et leurs mélanges, des agents épaississants minéraux comme notamment les argiles, des agents antioxydants tels que par exemple l'acide ascorbique, l'acide érythorbique, des agents réducteurs différents des composés (I) mentionnés précédemment tels que entre autres l'acide érythorbique, les sucres réducteurs, des agents de pénétration, des agents séquestrants comme l'éthylènediamine tétraacétique et ses sels, des parfums, des agents matifiants avec par exemple les oxydes de titane, des tampons, des agents dispersants, des agents filmogènes, des céramides et des agents conservateurs.

Les adjuvants ci-dessus sont en général présents en quantité comprise pour chacun d'eux entre 0,01 et 20 % en poids par rapport au poids de la composition.

La composition selon l'invention peut également comprendre un milieu cosmétiquement acceptable.

Le milieu cosmétiquement acceptable de la composition, qui est un milieu approprié pour la coloration des fibres kératiniques humaines, comprend de préférence de l'eau et éventuellement un ou plusieurs solvants organiques, différents des composés organiques (II) et différents des composés (I) mentionnés précédemment.

A titre d'exemples de tels solvants organiques, on peut par exemple citer, les alcanols, linéaires ou ramifiés, en $C_2$-$C_4$, tels que l'éthanol et l'isopropanol, les polyols et éthers de polyols comme le 2-butoxyéthanol, l'héxylène glycol, le propylèneglycol, le dipropylèneglycol, le glycérol et leurs mélanges.

Ce ou ces solvants organiques peuvent être présents dans des proportions allant de préférence de 1 à 40 % en poids par rapport au poids total de la composition tinctoriale, et encore plus préférentiellement de 5 à 30 % en poids.

La quantité d'eau dans la composition selon l'invention est de préférence supérieure à 10 % en poids par rapport au poids de la composition, et plus avantageusement supérieure ou égale à 25 % en poids. De préférence, la teneur en eau est comprise entre 25 et 98 % en poids par rapport au poids de la composition.

Le pH de la composition selon l'invention peut être compris entre 2 et 12, bornes comprises. De préférence, le pH de la composition selon l'invention est compris entre 8 et 12, bornes comprises.

Il peut être ajusté à la valeur désirée au moyen d'un ou plusieurs agents acidifiants.

Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

La composition selon l'invention peut comprendre également un ou plusieurs agents oxydants.

En particulier, la composition avec oxydant est obtenue par mélange extemporané avant l'application, d'une composition précédemment décrite, avec au moins une composition comprenant un ou plusieurs agents oxydants.

L'agent oxydant est choisi de préférence parmi le peroxyde d'hydrogène, le peroxyde d'urée, les bromates ou ferricyanures de métaux alcalins, les sels peroxygénés comme par exemple les persulfates, les perborates et les percarbonates de métaux alcalins et alcalino-terreux, comme le sodium, le potassium, le magnésium.

L'utilisation du peroxyde d'hydrogène est particulièrement préférée.

Cet agent oxydant est avantageusement constitué du peroxyde d'hydrogène en solution aqueuse (eau oxygénée) dont le titre peut varier, plus particulièrement, de 1 à 40 volumes, et encore plus préférentiellement de 5 à 40 volumes.

Les compositions selon l'invention peuvent résulter du mélange extemporané de plusieurs compositions.

Un autre objet de l'invention est donc constitué par un procédé de coloration de fibres kératiniques, notamment les fibres kératiniques humaines telles que les cheveux, qui consiste à appliquer la composition selon l'invention décrite précédemment.

Conformément à un premier mode de réalisation, la composition appliquée ne comprend pas d'agent oxydant. Ce mode de réalisation est approprié notamment dans le cas où la composition ne comprend pas de précurseur de colorant d'oxydation (bases, coupleurs).

Conformément à un deuxième mode de réalisation, la composition est appliquée en présence d'au moins un agent oxydant.

Ce mode de réalisation peut être mis en œuvre si la composition ne comprend, à titre de colorants, que des colorants directs ou colorant(s) à caractère hydrophobe, et éventuellement un ou plusieurs colorants directs additionnels, ou bien encore si la composition comprend un ou plusieurs colorants à caractère hydrophobe, éventuellement un ou plusieurs colorants directs additionnels de synthèse et/ou naturels, combinés à un ou plusieurs précurseurs de colorants d'oxydation (bases et coupleurs).

Selon une première variante de ce deuxième mode de réalisation, on applique sur les fibres la composition qui vient d'être détaillée et qui est obtenue par mélange extemporané avant l'application, d'une composition selon l'invention dépourvue d'agent oxydant avec une composition oxydante.

Selon une deuxième variante de ce deuxième mode de réalisation, on applique la composition selon l'invention dépourvue d'agent oxydant, et une composition oxydante, successivement et sans rinçage intermédiaire.

La composition oxydante mise en œuvre comprend un ou plusieurs agents oxydants tels que définis plus haut.

Concernant les solvants organiques éventuellement présents dans la composition oxydante, on pourra se

reporter à la liste indiquée auparavant dans le cadre du descriptif de la composition selon l'invention. Ces solvants organiques peuvent également être choisis parmi les composés organiques (II) présents dans la composition selon l'invention présentant une valeur du paramètre δH de Hansen de solubilité inférieure ou égale à 16 Mpa$^{1/2}$ et de poids moléculaire inférieur à 250 g/mol et différents des composés (I) mentionnés précédemment.

La composition oxydante peut se présenter sous la forme d'une solution, une émulsion ou un gel.

Elle peut éventuellement comprendre un ou plusieurs additifs utilisés classiquement dans le domaine de la coloration des fibres kératiniques humaines, en fonction de la forme galénique souhaitée. On pourra là encore se reporter à la liste des additifs donnée plus haut.

Quelle que soit le mode de réalisation retenu (avec ou sans oxydant), le mélange appliqué sur les fibres est laissé en place pour une durée, en général, de l'ordre de 1 minute à 1 heure, de préférence de 10 minutes à 30 minutes.

La température durant le procédé est classiquement comprise entre 10 et 200°C et plus particulièrement comprise entre la température ambiante (entre 15 à 25°C) et 80°C, de préférence entre la température ambiante et 60°C.

A l'issue du traitement, les fibres kératiniques humaines sont éventuellement rincées à l'eau, lavées au shampooing, rincées à nouveau à l'eau puis séchées ou laissées à sécher.

L'invention a également pour objet un dispositif à plusieurs compartiments ou "kit" de teinture, dans lequel un premier compartiment renferme la composition tinctoriale selon la présente invention telle que décrite ci-avant à l'exception du ou des agents oxydants, et un deuxième compartiment renferme une composition comprenant un ou plusieurs agents oxydants tels que décrits ci-avant.

Les exemples qui suivent sont destinés à illustrer l'invention sans pour autant en limiter la portée.

Dans ces exemples, toutes les quantités sont indiquées en pour cent en poids de matière active par rapport au poids total de la composition, sauf indication contraire.

<u>Exemple 1</u>

On prépare les compositions de coloration selon l'invention, dont les formulations sont données dans le tableau 1 ci-dessous.

Tableau 1

| | 1 | 2 | 3 | 4 | 5 |
|---|---|---|---|---|---|
| Indigo [1] | 1g | - | - | - | - |
| Isoindigo [2] | - | 1g | - | - | - |
| Pourpre de tyr [3] | - | - | 1g | - | - |
| Thioindigo [4] | - | - | - | 1g | - |
| Oralith [5] | - | - | - | - | 1g |
| Alcool benzylique | 5g | 5g | 5g | 5g | 5g |
| Ethanol | 15g | 15g | 15g | 15g | 15g |
| Acétoïne | 1g | 1g | 1g | 1g | 1g |
| Carbonate de sodium | 2g | 2g | 2g | 2g | 2g |
| Eau | qsp 100g | qsp 100g | qsp 100g | qsp 100g | qsp 100g |
| [1] Indigo, CAS=482-89-3, commercialisé par Sigma Aldrich<br>[2] Isoindigo<br>[3] Pourpre de tyr, 6,6'-dibromo indigo commercialisé par Kremer Pigmente<br>[4] Thioindigo, CAS=522-75-8, commercialisé par Sigma Aldrich<br>[5] Oralith, CAS=2379-74-0, commercialisé par Sigma Aldrich | | | | | |

Les compositions sont laissées agiter sous argon pendant 2H à 40°C.

Puis, le pH est ajusté à 9,6 avec 4 g d'ammoniac à 25% en solution aqueuse et 4 g d'acide lactique à 90% en solution aqueuse.

Des couples de mèches de cheveux à 90% de cheveux blancs naturels et permanentés sont colorées 30 minutes à température ambiante par ces compositions colorantes 1 à 5, les mèches sont ensuite essorées, rincées, shampouinées et séchées.

On obtient des colorations puissantes, et tenaces.

Exemple 2

On prépare les compositions de coloration selon l'invention, dont les formulations sont données dans le tableau 2 ci-dessous.

Tableau 2

| | 6 | 7 | 8 | 9 | 10 |
|---|---|---|---|---|---|
| Indigo [1] | 1g | - | - | - | - |
| Isoindigo [2] | - | 1g | - | - | - |
| Pourpre de tyr [3] | - | - | 1g | - | - |
| Thioindigo [4] | - | - | - | 1g | - |
| Oralith [5] | - | - | - | - | 1g |
| Alcool benzylique | 5g | 5g | 5g | 5g | 5g |
| Ethanol | 15g | 15g | 15g | 15g | 15g |
| Acétoïne | 1g | 1g | 1g | 1g | 1g |
| Hydrogénocarbonate de sodium | 2g | 2g | - | - | - |
| Hydrogénocarbonate de potassium | - | - | 2g | 2g | 2g |
| Eau | qsp 100g | qsp 100g | qsp 100g | qsp 100g | qsp 100g |
| [1] Indigo, CAS=482-89-3, commercialisé par Sigma Aldrich<br>[2] Isoindigo<br>[3] Pourpre de tyr, 6,6'-dibromo indigo commercialisé par Kremer Pigmente<br>[4] Thioindigo, CAS=522-75-8, commercialisé par Sigma Aldrich<br>[5] Oralith, CAS=2379-74-0, commercialisé par Sigma Aldrich | | | | | |

Les compositions sont laissées agiter sous argon pendant 2H à 40°C.

Puis, le pH est ajusté à 9,6 avec 4 g d'ammoniac à 25% en solution aqueuse et 4 g d'acide lactique à 90% en solution aqueuse.

Des couples de mèches de cheveux à 90% de cheveux blancs naturels et permanentés sont colorées 30 minutes à température ambiante par ces compositions colorantes 6 à 10, les mèches sont ensuite essorées, rincées, shampouinées et séchées.

On obtient des colorations puissantes, et tenaces.

Exemple 3

On prépare les compositions de coloration selon l'invention, dont les formulations sont données dans le tableau 3 ci-dessous.

Tableau 3

| | 11 | 12 | 13 |
|---|---|---|---|
| Indigo [1] | 1g | 1g | 1g |
| Glutaroïne | 1g | - | - |
| Dihydroxyacétone | - | 1g | - |
| Benzoïne | - | - | 1g |
| Alcool benzylique | 5g | 5g | 5g |
| Ethanol | 15g | 15g | 15g |
| Hydrogénocarbonate de sodium | 2g | 2g | 2g |

(suite)

| | 11 | 12 | 13 |
|---|---|---|---|
| Eau | qsp 100g | qsp 100g | qsp 100g |
| [1] Indigo, CAS=482-89-3, commercialisé par Sigma Aldrich | | | |

Les compositions sont préparées et appliquées sur des couples de mèches naturelles et permanentés de cheveux à 90% de cheveux blancs, dans les mêmes conditions que celles de l'exemple 2.
On obtient des colorations bleues puissantes, et tenaces.

Exemple 4

On prépare les compositions de coloration selon l'invention, dont les formulations sont données dans le tableau 4 ci-dessous.

Tableau 4

| | 14 | 15 | 16 |
|---|---|---|---|
| Indigo [1] | 0,5g | 0,5g | 0,5g |
| Curcumine | 0,5g | 0,5g | 0,5g |
| Isatine | 0,3g | 0,3g | 0,3g |
| Orceine | 0,3g | 0,3g | 0,3g |
| Chlorophyline | 0,15g | 0,15g | 0,15g |
| Sorgho | 0,02g | 0,02g | 0,02g |
| Acide laccaïque | 0,01g | 0,01g | 0,01g |
| Acétoïne | 1g | 1g | 1g |
| Ethanol | 15g | 15g | 15g |
| Alcool benzylique | 5g | 1g | - |
| 3-phényl-1-propanol | - | 0,5g | - |
| Décanol | - | - | 5g |
| CTAB [6] | - | - | 2g |
| Hydrogénocarbonate de sodium | 2g | 2g | 2g |
| Parfum | qs | qs | qs |
| Eau | qsp 100g | qsp 100g | qsp 100g |
| [1] Indigo, CAS=482-89-3, commercialisé par Sigma Aldrich<br>[6] Chlorure de cétyl triméthyl ammonium | | | |

Les compositions sont préparées et appliquées sur des couples de mèches naturelles et permanentés de cheveux à 90% de cheveux blancs, dans les mêmes conditions que celles de l'exemple 2.
On obtient des colorations marron puissantes, et tenaces.

Exemple 5

On prépare les compositions de coloration selon l'invention, dont les formulations sont données dans le tableau 5 ci-dessous. Les teneurs sont exprimées en grammes par rapport à 100 g de poids total de la composition.

Tableau 5

| | 17 | 18 | 19 | 20 | 21 |
|---|---|---|---|---|---|
| Indigo [1] | 1g | - | - | - | - |
| Isoindigo [2] | - | 1g | - | - | - |
| Pourpre de tyr [3] | - | - | 1g | - | - |
| Thioindigo [4] | - | - | - | 1g | - |
| Oralith [5] | - | - | - | - | 1g |
| Alcool benzylique | 5g | 5g | 5g | 5g | 5g |
| Ethanol | 15g | 15g | 15g | 15g | 15g |
| Acétoïne | 1g | 1g | 1g | 1g | 1g |
| Monoéthanolamine | 2g | 2g | 2g | 2g | 2g |
| Eau | qsp 100g | qsp 100g | qsp 100g | qsp 100g | qsp 100g |

[1] Indigo, CAS=482-89-3, commercialisé par Sigma Aldrich
[2] Isoindigo
[3] Pourpre de tyr, 6,6'-dibromo indigo commercialisé par Kremer Pigmente
[4] Thioindigo, CAS=522-75-8, commercialisé par Sigma Aldrich
[5] Oralith, CAS=2379-74-0, commercialisé par Sigma Aldrich

Les compositions sont laissées agiter sous argon pendant 2H à 40°C.

Puis, le pH est ajusté à 9,6 avec 4 g d'ammoniac à 25% en solution aqueuse et 4 g d'acide lactique à 90% en solution aqueuse.

Des couples de mèches de cheveux à 90% de cheveux blancs naturels et permanentés sont colorées 30 minutes à température ambiante par ces compositions colorantes 17 à 21, les mèches sont ensuite essorées, rincées, shampouinées et séchées.

On obtient des colorations puissantes, et tenaces.

Exemple 6

On prépare les compositions de coloration selon l'invention, dont les formulations sont données dans le tableau 6 ci-dessous. Les teneurs sont exprimées en grammes par rapport à 100 g de poids total de la composition.

Tableau 6

| | 22 | 23 | 24 | 25 | 26 |
|---|---|---|---|---|---|
| Indigo [1] | 1g | - | - | - | - |
| Isoindigo [2] | - | 1g | - | - | - |
| Pourpre de tyr [3] | - | - | 1g | - | - |
| Thioindigo [4] | - | - | - | 1g | - |
| Oralith [5] | - | - | - | - | 1g |
| Alcool benzylique | 5g | 5g | 5g | 5g | 5g |
| Ethanol | 15g | 15g | 15g | 15g | 15g |
| Acétoïne | 1g | 1g | 1g | 1g | 1g |
| Triéthanolamine | 2g | 2g | 2g | 2g | 2g |

(suite)

| | 22 | 23 | 24 | 25 | 26 |
|---|---|---|---|---|---|
| Eau | qsp 100g | qsp 100g | qsp 100g | qsp 100g | qsp 100g |
| [1] Indigo, CAS=482-89-3, commercialisé par Sigma Aldrich<br>[2] Isoindigo<br>[3] Pourpre de tyr, 6,6'-dibromo indigo commercialisé par Kremer Pigmente<br>[4] Thioindigo, CAS=522-75-8, commercialisé par Sigma Aldrich<br>[5] Oralith, CAS=2379-74-0, commercialisé par Sigma Aldrich | | | | | |

Les compositions sont laissées agiter sous argon pendant 2H à 40°C.

Puis, le pH est ajusté à 9,6 avec 4 g d'ammoniac à 25% en solution aqueuse et 4 g d'acide lactique à 90% en solution aqueuse.

Des couples de mèches de cheveux à 90% de cheveux blancs naturels et permanentés sont colorées 30 minutes à température ambiante par ces compositions colorantes 22 à 26, les mèches sont ensuite essorées, rincées, shampouinées et séchées.

On obtient des colorations puissantes, et tenaces.

Exemple 7

On prépare les compositions de coloration selon l'invention, dont les formulations sont données dans le tableau 7 ci-dessous. Les teneurs sont exprimées en grammes par rapport à 100 g de poids total de la composition.

Tableau 7

| | 27 | 28 | 29 |
|---|---|---|---|
| Indigo [1] | 1g | 1g | 1g |
| Glutaroïne | 1g | - | - |
| Dihydroxyacétone | - | 1g | - |
| Benzoïne | - | - | 1g |
| Alcool benzylique | 5g | 5g | 5g |
| Ethanol | 15g | 15g | 15g |
| Monoéthanolamine | 2g | 2g | 2g |
| Eau | qsp 100g | qsp 100g | qsp 100g |
| [1] Indigo, CAS=482-89-3, commercialisé par Sigma Aldrich | | | |

Les compositions sont préparées et appliquées sur des couples de mèches naturelles et permanentés de cheveux à 90% de cheveux blancs, dans les mêmes conditions que celles de l'exemple 6.

On obtient des colorations bleues puissantes, et tenaces.

Exemple 8

On prépare les compositions de coloration selon l'invention, dont les formulations sont données dans le tableau 8 ci-dessous. Les teneurs sont exprimées en grammes par rapport à 100 g de poids total de la composition.

Tableau 8

| | 30 | 31 | 32 |
|---|---|---|---|
| Indigo [1] | 0,5g | 0,5g | 0,5g |
| Curcumine | 0,5g | 0,5g | 0,5g |

(suite)

| | 30 | 31 | 32 |
|---|---|---|---|
| Isatine | 0,3g | 0,3g | 0,3g |
| Orceine | 0,3g | 0,3g | 0,3g |
| Chlorophyline | 0,15g | 0,15g | 0,15g |
| Sorgho | 0,02g | 0,02g | 0,02g |
| Acide laccaïque | 0,01g | 0,01g | 0,01g |
| Acétoïne | 1g | 1g | 1g |
| Ethanol | 15g | 15g | 15g |
| Alcool benzylique | 5g | 1g | - |
| 3-phényl-1-propanol | - | 0,5g | - |
| Décanol | - | - | 5g |
| CTAB [6] | - | - | 2g |
| Monoéthanolamine | 2g | 2g | 2g |
| Parfum | qs | qs | qs |
| Eau | qsp 100g | qsp 100g | qsp 100g |
| [1] Indigo, CAS=482-89-3, commercialisé par Sigma Aldrich<br>[6] Chlorure de cétyle triméthyle ammonium | | | |

Les compositions sont préparées et appliquées sur des couples de mèches naturelles et permanentés de cheveux à 90% de cheveux blancs, dans les mêmes conditions que celles de l'exemple 6.

On obtient des colorations marron puissantes, et tenaces.

Exemple 9

On prépare les compositions de coloration suivantes dont les formulations sont données dans le tableau 9. La composition 33 est une composition comparative. La composition 34 est une composition selon l'invention.

Tableau 9

| | 33 | 34 |
|---|---|---|
| Indigo [1] | 1g | 1g |
| Alcool benzylique | - | 5g |
| Ethanol | 20g | 15g |
| Acétoïne | 1g | 1g |
| Hydroxyde de sodium à 10% en solution aqueuse | 12g | 12g |
| Polyquaternium-7 à 8% en solution aqueuse | 3g | - |
| Eau | qsp 100g | qsp 100g |
| [1] Indigo, CAS=482-89-3, commercialisé par Sigma Aldrich | | |

Les compositions sont laissées agiter sous argon :

- pendant 4H à 40°C pour la solution 33
- pendant 2H à 40°C pour la solution 34

On note que la composition 33 n'est que partiellement solubilisée tandis que la composition 34 est parfaitement solubilisée.

Puis, le pH est ajusté à 9,6 avec 4 g d'ammoniac à 25% en solution aqueuse et 4 g d'acide lactique à 90% en solution aqueuse.

La solubilisation est nettement meilleure dans le cas de la composition 34 que dans le cas de la composition 33.

Exemple 10

On prépare les compositions de coloration selon l'invention suivantes dont les formulations sont données dans le tableau 10.

Tableau 10

| | 35 | 36 | 37 | 38 |
|---|---|---|---|---|
| Isoindigo [2] | 1g | 1g | 1g | 1g |
| Pourpre de tyr [3] | - | 1g | - | - |
| Thioindigo [4] | - | - | 1g | - |
| Oralith [5] | - | - | - | 1g |
| Alcool benzylique | 5g | 5g | 5g | 5g |
| Ethanol | 15g | 15g | 15g | 15g |
| Acétoïne | 1g | 1g | 1g | 1g |
| Sodium hydroxyde à 10% en solution aqueuse | 12g | 12g | 12g | 12g |
| Eau | qsp 100g | qsp 100g | qsp 100g | qsp 100g |
| [2] Isoindigo<br>[3] Pourpre de tyr, 6,6'-dibromo indigo commercialisé par Kremer Pigmente<br>[4] Thioindigo, CAS=522-75-8, commercialisé par Sigma Aldrich<br>[5] Oralith, CAS=2379-74-0, commercialisé par Sigma Aldrich | | | | |

Les compositions sont laissées agiter sous argon pendant 2H à 40°C.

Puis, le pH est ajusté à 9,6 avec 4 g d'ammoniac à 25% en solution aqueuse et 4 g d'acide lactique à 90% en solution aqueuse.

Des couples de mèches de cheveux à 90% de cheveux blancs naturels et permanentés sont colorées 20 minutes à 40°C par ces compositions colorantes 35 à 38, les mèches sont ensuite essorées, rincées, shampouinées et séchées.

On obtient des colorations puissantes, et tenaces.

Les résultats de coloration sur mèches sont observés visuellement et accompagnés de mesures colorimétriques exprimées en ΔE par rapport au cheveu non coloré.

Les mesures colorimétriques peuvent être réalisées à l'aide du spectrocolorimètre Konica Minolta CM-2600d dans le système CIE L*a*b*. Dans ce système, L* représente l'intensité de la coloration obtenue, plus la valeur de L* est faible, plus la coloration obtenue est intense. La chromaticité est mesurée par les valeurs a* et b*, a* indiquant la valeur sur l'axe de couleur vert/rouge et b* indiquant la valeur sur l'axe de couleur bleu/jaune.

La montée est mesurée par ΔE à partir de la formule :

$$\Delta E = \sqrt{(L^* - L_o{}^*)^2 + (a^* - a_o{}^*)^2 + (b^* - b_o{}^*)^2}$$

dans laquelle :

- L*, a* et b*, représentent les paramètres des cheveux colorés et
- $L_0$*, $a_0$* et $b_0$* représentent les paramètres des cheveux non colorés.

Ces mesures colorimétriques sont données dans le tableau 11.

Tableau 11

| | Montée sur cheveux naturels | | | | Montée sur cheveux permanentés | | | |
|---|---|---|---|---|---|---|---|---|
| | L* | a* | b* | ΔE/cheveu non coloré | L* | a* | b* | ΔE/cheveu non coloré |
| *Cheveu non coloré* | 60,38 | 0,57 | 13,09 | - | 61,38 | 0,31 | 12,77 | - |
| Cheveu coloré par 34 | 34,46 | -4,06 | -6,04 | 32,55 | 25,58 | -0,3 | -8,31 | 41,55 |
| Cheveu coloré par 35 | 30,93 | -3,46 | -7,26 | 36,02 | 27,55 | -1,98 | -8,79 | 40,18 |
| Cheveu coloré par 36 | 46,24 | 7,64 | 0,9 | 19,96 | 39,64 | 11,18 | -4,48 | 29,81 |
| Cheveu coloré par 37 | 35,4 | 7,11 | -1,23 | 29,53 | 26,93 | 9,48 | -5,01 | 39,85 |
| Cheveu coloré par 38 | 44,68 | 19,35 | 2,94 | 26,49 | 34,99 | 24,99 | -0,12 | 39,36 |

Exemple 11

On prépare les compositions de coloration selon l'invention suivantes dont les formulations sont données dans le tableau 12.

Tableau 12

| | 39 | 40 | 41 |
|---|---|---|---|
| Indigo [1] | 1g | 1g | 1g |
| Glutaroïne | 1g | - | - |
| Dihydroxyacétone | - | 1g | - |
| Benzoïne | - | - | 1g |
| Alcool benzylique | 5g | 5g | 5g |
| Ethanol | 15g | 15g | 15g |
| Sodium hydroxyde à 10% en solution aqueuse | 12g | 12g | 12g |
| Eau | qsp 100g | qsp 100g | qsp 100g |
| [1] Indigo, CAS=482-89-3, commercialisé par Sigma Aldrich | | | |

Les compositions sont préparées et appliquées sur des couples de mèches naturelles et permanentés de cheveux à 90% de cheveux blancs, dans les mêmes conditions que celles de l'exemple 10.

On obtient des colorations bleues puissantes, et tenaces.

Exemple 12

On prépare les compositions de coloration suivantes, dont les formulations sont données dans le tableau 13 ci-dessous. La composition 42 est une composition comparative. La composition 43 est une composition selon l'invention.

Tableau 13

| | 42 | 43 |
|---|---|---|
| Diosindigo A | 1g | 1g |

(suite)

| | 42 | 43 |
|---|---|---|
| Alcool benzylique | 5g | 5g |
| Ethanol | 15g | 15g |
| Acétoïne | - | 1g |
| Hydroxyde de sodium à 10% en solution aqueuse | 12g | 12g |
| Eau | qsp 100g | qsp 100g |

Les compositions sont laissées agiter sous argon pendant 30 minutes à température ambiante.

Puis le pH de chaque composition est ajusté à 9,6 avec 4 g d'ammoniac à 20% en solution aqueuse et 4 g d'acide lactique à 90% en solution aqueuse.

Des couples de mèches de cheveux à 90% de cheveux blancs naturels et permanentés sont alors colorés au moyen de chacune des compositions 42 et 43. Les compositions sont appliquées sur les mèches, puis après un temps de pose de 30 minutes à température ambiante, les mèches sont rincées, shampouinées et séchées.

On constate alors que l'on n'obtient que peu de coloration pour les mèches de cheveux (naturels et permanentés) colorées au moyen de la composition 42, tandis que l'on obtient une coloration bleue intense pour les mèches de cheveux (naturels et permanentés) colorées au moyen de la composition 43.

Les mesures colorimétriques ont été réalisées à l'aide du spectrocolorimètre Konica Minolta CM-2600d dans le système CIE L*a*b*.

Selon ce système, L* représente l'intensité de la coloration. Les coordonnées chromatiques sont exprimées par les paramètres a* et b*, a* correspondant à l'axe chromatique rouge / vert et b* à l'axe chromatique jaune / bleu.

La montée de la couleur ΔE, qui est l'écart de couleur entre les mèches non colorées et les mèches colorées, est déterminée selon l'équation suivante :

$$\Delta E = \sqrt{(L^* - L_o^*)^2 + (a^* - a_o^*)^2 + (b^* - b_o^*)^2}$$

Dans cette équation, L*, a* et b* représentent les valeurs mesurées sur les mèches de cheveux colorées, et $L_0$*, $a_0$* et $b_0$* représentent les valeurs mesurées sur les mèches de cheveux non colorées.

Plus la valeur de ΔE est importante, plus la différence de couleur entre les mèches colorées et non colorées est importante, ce qui est représentatif d'une meilleure montée de la couleur sur les mèches de cheveux.

Les résultats obtenus sont les suivants :

| | 42 | 43 |
|---|---|---|
| Montée sur cheveux naturels | 5.2 | 32.85 |
| Montée sur cheveux permanentés | 12.52 | 40.56 |

Ces résultats confirment qu'avec la composition 43 selon l'invention, l'on obtient une montée de la couleur sur les cheveux, tant naturels que permanentés, nettement supérieure par rapport à la composition comparative 42.

On obtient également, avec la composition B selon l'invention, une valeur plus basse du paramètre L*, ce qui traduit une meilleure intensité de la coloration.

Exemple 14

On prépare les compositions de coloration selon l'invention, dont les formulations sont données dans le tableau 15 ci-dessous.

Tableau 15

| | 46 | 47 | 48 |
|---|---|---|---|
| Diosindigo A | 1g | 1g | 1g |

(suite)

| | 46 | 47 | 48 |
|---|---|---|---|
| Glutaroïne | 1g | - | - |
| Dihydroxyacétone | - | 1g | - |
| Benzoïne | - | - | 1g |
| Alcool benzylique | 5g | 5g | 5g |
| Ethanol | 15g | 15g | 15g |
| Hydroxyde de sodium à 10% en solution aqueuse | 12g | 12g | 12g |
| Eau | qsp 100g | qsp 100g | qsp 100g |

Les compositions sont laissées agiter sous argon à température ambiante, jusqu'à dissolution des ingrédients.
Puis le pH de chacune des trois compositions est ajusté à 9,6 avec 4 g d'ammoniac à 20% en solution aqueuse et 4 g d'acide lactique à 90% en solution aqueuse.
Des couples de mèches de cheveux à 90% de cheveux blancs naturels et permanentés sont alors colorés au moyen de chacune des compositions 46, 47 et 48. Les compositions sont appliquées sur les mèches, puis après un temps de pose de 30 minutes à température ambiante, les mèches sont rincées, shampouinées et séchées.
On constate alors que l'on obtient, avec chacune de ces trois compositions, des colorations bleues, puissantes et tenaces.

Exemple 15

On prépare les compositions de coloration selon l'invention, dont les formulations sont données dans le tableau 16 ci-dessous.

Tableau 16

| | 49 | 50 | 51 |
|---|---|---|---|
| Diosindigo A | 0,5 g | 0,5 g | 0,5 g |
| Curcumine | 0,5 g | 0,5 g | 0 ,5 g |
| Isatine | 0,3 g | 0,3 g | 0,3 g |
| Orceïne | 0,3 g | 0,3 g | 0,3 g |
| Chlorophyline | 0,15 g | 0,15 g | 0,15 g |
| Sorgho | 0,02 g | 0,02 g | 0,02 g |
| Acide laccaïque | 0,01 g | 0,01 g | 0,01 g |
| Acétoïne | 1 g | 1 g | 1 g |
| Ethanol | 15 g | 15 g | 15 g |
| Alcool benzylique | 5 g | 1 g | - |
| 3-phényl-1-propanol | - | 0,5 g | - |
| Décanol | - | - | 5 g |
| Chlorure de cétyl triméthyl ammonium | - | - | 2g |
| Hydroxyde de sodium à 10% en solution aqueuse | 12g | 12g | 12g |
| Parfum | qs | qs | qs |
| Eau | qsp 100 | qsp 100 | qsp 100 |

Les compositions sont laissées agiter sous argon à température ambiante, jusqu'à dissolution des ingrédients.
Puis le pH de chacune des trois compositions est ajusté à 9,6 avec 4 g d'ammoniac à 20% en solution aqueuse

et 4 g d'acide lactique à 90% en solution aqueuse.

Des couples de mèches de cheveux à 90% de cheveux blancs naturels et permanentés sont alors colorés au moyen de chacune des compositions 49, 50 et 51. Les compositions sont appliquées sur les mèches, puis après un temps de pose de 30 minutes à température ambiante, les mèches sont rincées, shampouinées et séchées.

On constate alors que l'on obtient, avec chacune de ces trois compositions, des colorations naturelles, puissantes et tenaces.

## Revendications

1. Composition tinctoriale comprenant :

   - un ou plusieurs colorants directs hydrophobes de logP supérieur ou égal à 2, choisis parmi les colorants indigoïdes,
   - un ou plusieurs agents alcalins organiques, et/ou une ou plusieurs bases minérales choisies parmi les carbonates, les hydrogénocarbonates, l'hydroxyde de sodium, l'hydroxyde de potassium, et leurs mélanges,
   - un ou plusieurs composés (I) comprenant dans leur structure un enchaînement :

   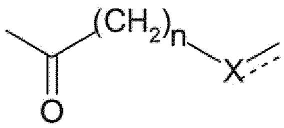

   avec n désignant un nombre entier allant de 0 à 4, et 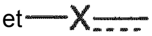 désignant un groupement :

   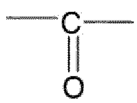 ou 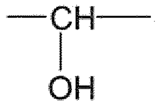 ou 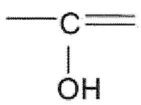 ,

   et
   - un ou plusieurs composés organiques (II) présentant une valeur du paramètre de solubilité $\delta H$ de Hansen compris entre 5 et 15 $Mpa^{1/2}$ et de poids moléculaire inférieur à 250 g/mol.

2. Composition selon la revendication 1 **caractérisée en ce que** :

   - les colorants indigoïdes sont choisis parmi l'indigo, l'isoindigo, l'indirubine, l'isoindirubine, le 4,4'-dibromo indigo, le 6,6'-dibromo indigo, le 5,5'-dibromo indigo, le cis-6,6'-dibromo indigo, le 5,5',7,7-tétrabromo indigo, le 4,4',7,7'-tétrachloroindigo, le 3H-indol-3-one, 1,2-dihydro-2-(3-oxobenzo[b]thien-2-(3H)-ylidène), le thioindigo, le Vat Red 1, le cis-thioindigo, le 6,6'-dichloro-4,4'-diméthylindigo, le 5,5'-dichloro-7,7'-diméthylindigo, le 4,4'-7,7'-tétraméthylindigo, le thioindigo Scarlet R, le 2H-indol-2-one, 1,3-dihydro-3-(3-oxobenzo[b]thien-2(3H)-ylidène)-, (3E)-, la thioindirubine, le 2H-indol-2-one, 1,3-dihydro-3-(2-oxobenzo[b]thiophen-3(2H)-ylidène), le benzo[b]thiophen-2(3H)-one, 3-(2-oxobenzo[b]thiophen-3(2H)-ylidène.

3. Composition selon l'une quelconque des revendications précédentes **caractérisée en ce que** le ou les colorants directs hydrophobes représentent de 0,001 à 20 % en poids, de préférence de 0,01 à 20 % en poids, préférentiellement de 0,01 à 10 % en poids, encore plus préférentiellement de 0,05 à 10 % en poids, mieux de 0,1 à 5 % en poids du poids total de la composition cosmétique.

4. Composition selon l'une quelconque des revendications précédentes **caractérisée en ce que** le ou les composés (I) présentent dans leur structure un enchaînement :

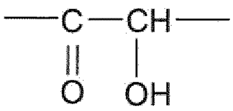 (A) ou 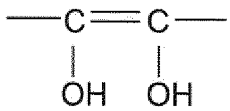 (B).

5. Composition selon l'une quelconque des revendications précédentes **caractérisée en ce que** le ou les composés (I) répondent à la formule (C) suivante :

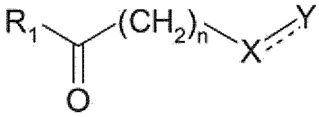

(C)

avec :

n désignant un nombre entier allant de 0 à 4, de préférence de 0 à 2, et 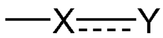 désignant un groupement :

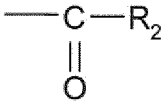 ou 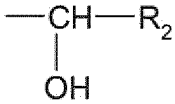 ou 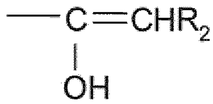 ,

$R_1$ et $R_2$ représentant, indépendamment l'un de l'autre, un atome d'hydrogène, un radical phényle substitué ou non substitué, un radical hydroxy, un radical alcoxy en $C_1$-$C_4$, un radical alkyle en $C_1$-$C_4$ linéaire ou ramifié, non substitué ou substitué par un ou deux radicaux choisis parmi les radicaux -OR', -C(O)R", -COOR''', avec R', R" et R''' représentant, indépendamment les uns des autres, un atome d'hydrogène, un radical alkyle en $C_1$-$C_4$ linéaire ou ramifié, ou

$R_1$ et $R_2$ formant, avec la chaîne carbonée à laquelle ils sont attachés, un cycle non aromatique carboné à 5 ou 6 chaînons non substitué,

ce ou ces composés (I) étant de préférence choisis parmi l'hydroxyacétone, l'acétoïne, la glutaroïne, l'adipoïne, la dihydroxyacétone, le glycol aldéhyde, la benzoïne, le 2,3-dihydroxyacrylaldéhyde, le cyclopentanedione, l'acétonyl acétone, l'acétyl acétone, le diacétyle, le dipropionyle, l'acide 2-cétoglutarique, l'acide 3-cétoglutarique, l'acide pyruvique, l'acide lévulinique, l'acide acétoacétique, l'acide propionylacétique, l'acide acétonylmalonique, l'acétate d'acétométhyle, l'acétate d'acétoéthyle, l'acide acétopyruvique, le cyclohexanedione.

6. Composition selon l'une quelconque des revendications précédentes **caractérisée en ce que** le ou les composés (I) représentent de 0,01 à 20 % en poids, de préférence de 0,05 à 10 % en poids, mieux de 0,1 à 5 % en poids du poids total de la composition cosmétique.

7. Composition selon l'une quelconque des revendications précédentes **caractérisée en ce que** :

- le ou les agents alcalins organiques présentent un $pK_b$ à 25° C allant de 1 à 12, de préférence de 1 à 10, et plus préférentiellement de 2 à 6,
- la ou les bases minérales sont choisies parmi les carbonates de métaux alcalins, les hydrogénocarbonates de métaux alcalins, et leurs mélanges, de préférence parmi le carbonate de sodium, le carbonate de potassium, l'hydogénocarbonate de sodium, l'hydrogénocarbonate de potassium, et leurs mélanges.

8. Composition selon l'une quelconque des revendications précédentes **caractérisée en ce que** le ou les agents alcalins organiques sont choisis parmi les amines organiques, de préférence choisis parmi :

(1) les amines comprenant une fonction amine primaire, secondaire ou tertiaire, et un ou plusieurs groupements alkyle, linéaires ou ramifiés, en $C_1$-$C_8$ porteurs d'un ou plusieurs radicaux hydroxyle,
(2) les amines organiques de formule suivante :

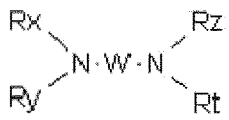

dans laquelle W est un reste alkylène en $C_1$-$C_6$ éventuellement substitué par un groupement hydroxyle ou un radical alkyle en $C_1$-$C_6$ ; Rx, Ry, Rz et Rt, identiques ou différents, représentent un atome d'hydrogène, un radical alkyle en $C_1$-$C_6$ ou hydroxyalkyle en $C_1$-$C_6$, aminoalkyle en $C_1$-$C_6$,
(3) les acides aminés,
(4) les amines organiques de type hétérocyclique,
(5) les dipeptides d'acides aminés,
(6) les composés comportant une fonction guanidine, et encore plus préférentiellement parmi les alcanolamines, les acides aminés basiques, les composés comportant une fonction guanidine, et leurs mélanges, et mieux parmi les alcanolamines, en particulier la nonoéthanolamine, la diéthanolamine et la triéthanolamine.

**9.** Composition selon l'une quelconque des revendications précédentes **caractérisée en ce que** le ou les agents alcalins organiques et/ou la ou les bases minérales représentent de 0,1 à 20% en poids, de préférence de 0,5 à 10% en poids, et plus préférentiellement de 1 à 5% en poids, par rapport au poids total de la composition.

**10.** Composition selon l'une quelconque des revendications précédentes **caractérisée en ce que** le ou les composés organiques (II) présentent une valeur du paramètre de solubilité $\delta$H de Hansen comprise entre 5 et 15 Mpa$^{1/2}$, plus préférentiellement entre 8 et 15 Mpa$^{1/2}$, ce ou ces composés étant présents à une teneur allant de 0,1 à 20%, de préférence de 1 à 5% en poids, du poids total de la composition.

**11.** Composition selon la revendication précédente, **caractérisée en ce que** le ou les composés organiques (II) sont choisis parmi les alcanols, les esters aliphatiques, les éthers, les alcools aromatiques, les alcools alkylaryle, les acides aromatiques, les acides aliphatiques, les carbonates d'alkylène, les lactones, et leurs mélanges, et de préférence parmi le 1-octanol, le 1-décanol, le tridécyl alcool, le dipropylène glycol monométhyléther acétate, le dipropylène glycol méthyléther, le tripropylène glycol méthyléther, le propylène glycol n-butyl éther, le propylène glycol n-propyl éther, le monométhyléther de propylèneglycol, le monoéthyléther et le monométhyléther du diéthylèneglycol, l'éthylène glycol 2-éthyl hexyl éther, le 3-phényl-1-propanol, le 2-phényl-1-propanol, l'alcool benzylique, le benzyloxyéthanol, le phénoxyéthanol, et leurs mélanges.

**12.** Composition selon l'une quelconque des revendications précédentes **caractérisée en ce que** la composition comprend :

- un ou plusieurs colorants additionnels, différents des colorants à caractère hydrophobe de logP supérieur ou égal à 2, choisis parmi les colorants naturels et les colorants directs non naturels, les précurseurs de colorant d'oxydation, et leurs combinaisons, et/ou
- un ou plusieurs agents oxydants, de préférence le peroxyde d'hydrogène.

**13.** Procédé de coloration consistant à appliquer sur les fibres kératiniques, notamment les fibres kératiniques humaines telles que les cheveux, une composition telle que définie dans l'une quelconque des revendications précédentes.

**Patentansprüche**

**1.** Färbezusammensetzung, umfassend:

- einen oder mehrere hydrophobe Direktfarbstoffe mit einem logP größer oder gleich 2, die aus Indigoid-Farbstoffen ausgewählt sind,
- ein oder mehrere organische alkalische Mittel und/oder eine oder mehrere anorganische Basen, die aus Carbonaten, Hydrogencarbonaten, Natriumhydroxid, Kaliumhydroxid und Mischungen davon ausgewählt sind,
- eine oder mehrere Verbindungen (I) die in ihrer Struktur eine Sequenz:

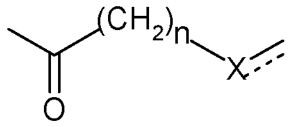

umfassen, wobei n für eine ganze Zahl im Bereich von 0 bis 4 steht und

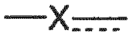 für eine Gruppe:

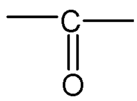 oder 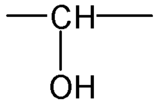 oder 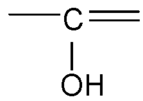

steht, und

- eine oder mehrere organische Verbindungen (II) mit einem Wert des Hansen-Löslichkeitsparameters δH zwischen 5 und 15 $MPa^{1/2}$ und mit einem Molekulargewicht von weniger als 250 g/mol.

2. Zusammensetzung nach Anspruch 1, **dadurch gekennzeichnet, dass**

- die Indigoid-Farbstoffe aus Indigo, Isoindigo, Indirubin, Isoindirubin, 4,4'-Dibromindigo, 6,6'-Dibromindigo, 5,5'-Dibromindigo, cis-6,6'-Dibromindigo, 5,5',7,7'-Tetrabromindigo, 4,4',7,7'-Tetrachlorindigo, 1,2-Dihydro-2-(3-oxobenzo[b]-thien-2-(3H)-yliden)-3H-indol-3-on, Thioindigo, Vat Red 1, cis-Thioindigo, 6,6'-Dichlor-4,4'-dimethylindigo, 5,5'-Dichlor-7,7'-dimethylindigo, 4,4'-7,7'-Tetramethylindigo, Thioindigo Scarlet R, (3E)-1,3-Dihydro-3-(3-oxobenzo[b]thien-2(3H)-yliden)-2H-indol-2-on, Thioindirubin, 1,3-Dihydro-3-(2-oxobenzo[b]thiophen-3(2H)-yliden)-2H-indol-2-on und 3-(2-Oxobenzo[b]thiophen-3(2H)-yliden-benzo[b]thiophen-2(3H)-on ausgewählt sind.

3. Zusammensetzung nach einem der vorhergehenden Ansprüche, **dadurch gekennzeichnet, dass** der hydrophobe Direktfarbstoff bzw. die hydrophoben Direktfarbstoffe 0,001 bis 20 Gew.-%, vorzugsweise 0,01 bis 20 Gew.-%, bevorzugt 0,01 bis 10 Gew.-%, noch weiter bevorzugt 0,5 bis 10 Gew.-% und noch besser 0,1 bis 5 Gew.-% des Gesamtgewichts der kosmetischen Zusammensetzung ausmacht bzw. ausmachen.

4. Zusammensetzung nach einem der vorhergehenden Ansprüche, **dadurch gekennzeichnet, dass** die Verbindung bzw. die Verbindungen (I) in ihrer Struktur eine Sequenz:

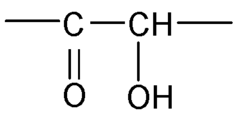 (A) oder 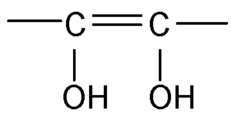 (B)

aufweist bzw. aufweisen.

5. Zusammensetzung nach einem der vorhergehenden Ansprüche, **dadurch gekennzeichnet, dass** die Verbindung bzw. die Verbindungen (I) der folgenden Formel (C) entspricht bzw. entsprechen:

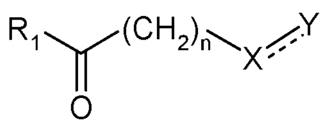

(C)

wobei:

n für eine ganze Zahl im Bereich von 0 bis 4 und vorzugsweise von 0 bis 2 steht und

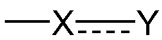 für eine Gruppe:

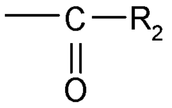 oder 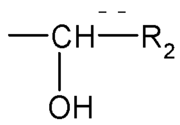 oder 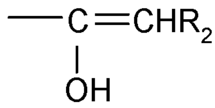

steht,

$R_1$ und $R_2$ unabhängig voneinander für ein Wasserstoffatom, einen substituierten oder unsubstituierten Phenylrest, einen Hydroxylrest, eine $C_1$-$C_4$-Alkoxyrest, einen linearen oder verzweigten $C_1$-$C_4$-Alkylrest, der unsubstituiert oder durch einen oder zwei Reste, die aus -OR', - C(O)R", -COR''' ausgewählt sind, substituiert ist, wobei R', R" und R''' unabhängig voneinander für ein Wasserstoffatom oder einen linearen oder verzweigten $C_1$-$C_4$-Alkylrest stehen, oder

$R_1$ und $R_2$ mit der Kohlenstoffkette, an die sie gebunden sind, einen unsubstituierten nichtaromatischen 5- oder 6-gliedrigen Kohlenstoffring bilden,

wobei diese Verbindung bzw. Verbindungen (I) vorzugsweise aus Hydroxyaceton, Acetoin, Glutaroin, Adipoin, Dihydroxyaceton, Glykolaldehyd, Benzoin, 2,3-Dihydroxyacrylaldehyd, Cyclopentandion, Acetonylaceton, Acetylaceton, Diacetyl, Dipropionyl, 2-Ketoglutarsäure, 3-Ketoglutarsäure, Brenztraubensäure, Lävulinsäure, Acetessigsäure, Propionylessigsäure, Acetonylmalonsäure, Acetessigsäuremethylester, Acetessigsäureethylester, Acetbrenztraubensäure und Cyclohexandion ausgewählt ist bzw. sind.

**6.** Zusammensetzung nach einem der vorhergehenden Ansprüche, **dadurch gekennzeichnet, dass** die Verbindung bzw. die Verbindungen (I) 0,01 bis 20 Gew.-%, vorzugsweise 0,05 bis 10 Gew.-% und noch besser 0,1 bis 5 Gew.-% des Gesamtgewichts der kosmetischen Zusammensetzung ausmacht bzw. ausmachen.

**7.** Zusammensetzung nach einem der vorhergehenden Ansprüche, **dadurch gekennzeichnet, dass**:

- das organische alkalische Mittel bzw. die alkalischen organischen Mittel einen $pK_b$-Wert bei 25 °C im Bereich von 1 bis 12, vorzugsweise 1 bis 10 und weiter bevorzugt von 2 bis 6 aufweist bzw. aufweisen,
- die anorganische Base bzw. die anorganischen Basen aus Alkalimetallcarbonaten, Alkalimetallhydrogencarbonaten und Mischungen davon, vorzugsweise aus Natriumcarbonat, Kaliumcarbonat, Natriumhydrogencarbonat, Kaliumhydrogencarbonat und Mischungen davon, ausgewählt ist bzw. sind.

**8.** Zusammensetzung nach einem der vorhergehenden Ansprüche, **dadurch gekennzeichnet, dass** das organische alkalische Mittel bzw. die organischen alkalischen Mittel aus organischen Aminen ausgewählt ist bzw. sind, vorzugsweise aus:

(1) Aminen mit einer primären, sekundären oder tertiären Aminfunktion und einer oder mehreren linearen oder verzweigten $C_1$-$C_8$-Alkylgruppen, die einen oder mehrere Hydroxylreste tragen,
(2) organischen Aminen der folgenden Formel:

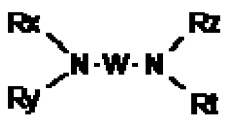

in der W für einen $C_1$-$C_6$-Alkylenrest, der gegebenenfalls durch eine Hydroxylgruppe oder einen $C_1$-$C_6$-Alkylrest substituiert ist, steht und Rx, Ry, Rz und Rt gleich oder verschieden sind und für ein Wasserstoffatom oder einen $C_1$-$C_6$-Alkyl-, $C_1$-$C_6$-Hydroxyalkyl- oder $C_1$-$C_6$-Aminoalkylrest stehen,
(3) Aminosäuren,
(4) organischen Aminen vom heterocyclischen Typ,
(5) Aminosäure-Dipeptiden,
(6) Verbindungen mit einer Guanidinfunktion, und noch weiter bevorzugt aus Alkanolaminen, basischen Aminosäuren, Verbindungen mit einer Guanidinfunktion und Mischungen davon und noch besser aus Alkanolaminen, insbesondere Monoethanolamin, Diethanolamin und Triethanolamin.

**9.** Zusammensetzung nach einem der vorhergehenden Ansprüche, **dadurch gekennzeichnet, dass** das organische alkalische Mittel bzw. die organischen alkalischen Mittel und/oder die anorganische Base bzw. die anorganischen

Basen 0,1 bis 20 Gew.-%, vorzugsweise 0,5 bis 10 Gew.-% und weiter bevorzugt 1 bis 5 Gew.-%, bezogen auf das Gesamtgewicht der Zusammensetzung, ausmachen.

**10.** Zusammensetzung nach einem der vorhergehenden Ansprüche, **dadurch gekennzeichnet, dass** die organischen Verbindungen (II) einen Wert des Hansen-Löslichkeitsparameters $\delta H$ zwischen 5 und 15 $MPa^{1/2}$ und weiter bevorzugt zwischen 8 und 15 $MPa^{1/2}$ aufweisen, wobei diese Verbindung bzw. diese Verbindungen in einem Gehalt im Bereich von 0,1 bis 20 Gew.-%, vorzugsweise 1 bis 5 Gew.-%, des Gesamtgewichts der Zusammensetzung vorliegt bzw. vorliegen.

**11.** Zusammensetzung nach dem vorhergehenden Anspruch, **dadurch gekennzeichnet, dass** die organische Verbindung bzw. die organischen Verbindungen (II) aus Alkanolen, aliphatischen Estern, Ethern, aromatischen Alkoholen, Alkylarylalkoholen, aromatischen Säuren, aliphatischen Säuren, Alkylencarbonaten, Lactonen und Mischungen davon und vorzugsweise aus 1-Octanol, 1-Decanol, Tridecylalkohol, Dipropylenglykolmonomethyletheracetat, Dipropylenglykolmethylether, Tripropylenglykolmethylether, Propylenglykol-n-butylether, Propylenglykol-n-propylether, Propylenglykolmonomethylether, Diethylenglykolmonomethylether und -monoethylether, Ethylenglykol-2-ethylhexylether, 3-Phenyl-1-propanol, 2-Phenyl-1-propanol, Benzylalkohol, Benzyloxyethanol und Phenoxyethanol und Mischungen davon ausgewählt ist bzw. sind.

**12.** Zusammensetzung nach einem der vorhergehenden Ansprüche, **dadurch gekennzeichnet, dass** die Zusammensetzung Folgendes umfasst:

- einen oder mehrere zusätzliche Farbstoffe, die von Farbstoffen mit hydrophobem Charakter mit einem logP-Wert größer oder gleich 2 verschieden sind und aus natürlichen Farbstoffen und nicht natürlichen Direktfarbstoffen, Oxidationsfarbstoff-Vorstufen und Kombinationen davon ausgewählt sind, und/oder
- ein oder mehrere Oxidationsmittel, vorzugsweise Wasserstoffperoxid.

**13.** Färbeverfahren, das darin besteht, dass man auf Keratinfasern, insbesondere menschliche Keratinfasern wie das Haar, eine Zusammensetzung gemäß einem der vorhergehenden Ansprüche aufbringt.

**Claims**

**1.** Dye composition comprising:

- one or more hydrophobic direct dyes with a logP of greater than or equal to 2, chosen from indigoid dyes,
- one or more organic alkaline agents, and/or one or more mineral bases chosen from carbonates, hydrogen carbonates, sodium hydroxide and potassium hydroxide, and mixtures thereof,
- one or more compounds (I) comprising in their structure a sequence:

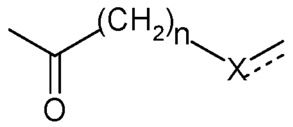

with n denoting an integer ranging from 0 to 4, and

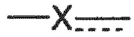 denoting a group:

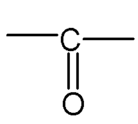 or 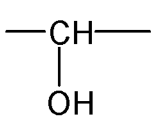 or 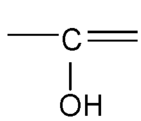,

and

- one or more organic compounds (II) with a Hansen solubility parameter $\delta H$ of between 5 and 15 $MPa^{1/2}$ and with a molecular weight of less than 250 g/mol.

**2.** Composition according to Claim 1, **characterized in that**:

- the indigoid dyes are chosen from indigo, isoindigo, indirubin, isoindirubin, 4,4'-dibromoindigo, 6,6'-dibromoindigo, 5,5'-dibromoindigo, cis-6,6'-dibromoindigo, 5,5',7,7'-tetrabromoindigo, 4,4',7,7'-tetrachloroindigo, 3H-indol-3-one, 1,2-dihydro-2-(3-oxobenzo[b]thien-2-(3H)-ylidene), thioindigo, Vat Red 1, cis-thioindigo, 6,6'-dichloro-4,4'-dimethylindigo, 5,5'-dichloro-7,7'-dimethylindigo, 4,4'-7,7'-tetramethylindigo, thioindigo Scarlet R, 2H-indol-2-one, 1,3-dihydro-3-(3-oxobenzo[b]thien-2(3H)-ylidene)-, (3E)-, thioindirubin, 2H-indol-2-one, 1,3-dihydro-3-(2-oxobenzo[b]thiophen-3(2H)-ylidene), benzo[b]thiophen-2(3H)-one and 3-(2-oxobenzo[b]thiophen-3(2H)-ylidene.

3. Composition according to either one of the preceding claims, **characterized in that** the hydrophobic direct dye(s) represent from 0.001% to 20% by weight, preferably from 0.01% to 20% by weight, preferentially from 0.01% to 10% by weight, even more preferentially from 0.05% to 10% by weight and better still from 0.1% to 5% by weight relative to the total weight of the cosmetic composition.

4. Composition according to any one of the preceding claims, **characterized in that** the compound(s) (I) have in their structure a sequence:

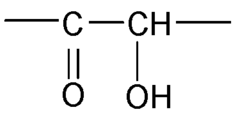 (A) or 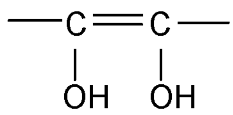 (B).

5. Composition according to any one of the preceding claims, **characterized in that** the compound(s) (I) correspond to formula (C) below:

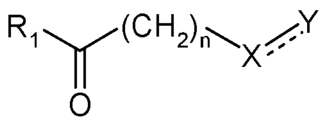

(C)

with:

n denoting an integer from 0 to 4 and preferably from 0 to 2, and

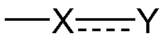 denoting a group:

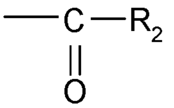 or 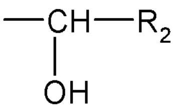 or 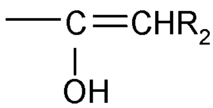 ,

$R_1$ and $R_2$ representing, independently of each other, a hydrogen atom; a substituted or unsubstituted phenyl radical; a hydroxyl radical; a $C_1$-$C_4$ alkoxy radical; a linear or branched $C_1$-$C_4$ alkyl radical, which is unsubstituted or substituted with one or two radicals chosen from the radicals -OR',-C(O)R" and -COOR"', with R', R" and R"' representing, independently of each other, a hydrogen atom or a linear or branched $C_1$-$C_4$ alkyl radical, or
$R_1$ and $R_2$ forming, with the carbon-based chain to which they are attached, an unsubstituted 5- or 6-membered nonaromatic carbon-based ring,
these compounds (I) preferably being chosen from hydroxyacetone, acetoin, glutaroin, adipoin, dihydroxyacetone, glycol aldehyde, benzoin, 2,3-dihydroxyacrylaldehyde, cyclopentanedione, acetonylacetone, acetylacetone, diacetyl, dipropionyl, 2-ketoglutaric acid, 3-ketoglutaric acid, pyruvic acid, levulinic acid, acetoacetic acid, propionylacetic acid, acetonylmalonic acid, methyl acetoacetate, ethyl acetoacetate, acetopyruvic acid and cyclohexanedione.

6. Composition according to any one of the preceding claims, **characterized in that** the compound(s) (I) represent from 0.01% to 20%, preferably from 0.05% to 10% and better still from 0.1% to 5% by weight relative to the total weight of the cosmetic composition.

7. Composition according to any one of the preceding claims, **characterized in that**:

- the organic alkaline agent(s) have a $pK_b$ at 25°C ranging from 1 to 12, preferably from 1 to 10 and more preferentially from 2 to 6,
- the mineral base(s) are chosen from alkali metal carbonates and alkali metal hydrogen carbonates and mixtures thereof, preferably from sodium carbonate, potassium carbonate, sodium hydrogen carbonate and potassium hydrogen carbonate, and mixtures thereof.

8. Composition according to any one of the preceding claims, **characterized in that** the organic alkaline agent(s) are chosen from organic amines, preferably chosen from:

(1) amines comprising a primary, secondary or tertiary amine function, and one or more linear or branched $C_1$-$C_8$ alkyl groups bearing one or more hydroxyl radicals,
(2) the organic amines of the following formula:

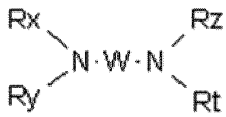

in which W is a $C_1$-$C_6$ alkylene residue optionally substituted with a hydroxyl group or a $C_1$-$C_6$ alkyl radical; Rx, Ry, Rz and Rt, which may be identical or different, represent a hydrogen atom or a $C_1$-$C_6$ alkyl, $C_1$-$C_6$ hydroxyalkyl or $C_1$-$C_6$ aminoalkyl radical,
(3) amino acids,
(4) organic amines of heterocyclic type,
(5) amino acid dipeptides,
(6) compounds comprising a guanidine function, and
even more preferentially from alkanolamines, basic amino acids, compounds comprising a guanidine function, and mixtures thereof, and better still from alkanolamines, in particular monoethanolamine, diethanolamine and triethanolamine.

9. Composition according to any one of the preceding claims, **characterized in that** the organic alkaline agent(s) and/or the mineral base(s) represent from 0.1% to 20% by weight, preferably from 0.5% to 10% by weight and more preferentially from 1% to 5% by weight relative to the total weight of the composition.

10. Composition according to any one of the preceding claims, **characterized in that** the organic compound(s) (II) have a Hansen solubility parameter $\delta H$ of between 5 and 15 $MPa^{1/2}$, more preferentially between 8 and 15 $MPa^{1/2}$, these compound(s) being present in a content ranging from 0.1% to 20% and preferably from 1% to 5% by weight relative to the total weight of the composition.

11. Composition according to the preceding claim, **characterized in that** the organic compound(s) (II) are chosen from alkanols, aliphatic esters, ethers, aromatic alcohols, alkylaryl alcohols, aromatic acids, aliphatic acids, alkylene carbonates, and lactones, and mixtures thereof, and preferably from 1-octanol, 1-decanol, tridecyl alcohol, dipropylene glycol monomethyl ether acetate, dipropylene glycol methyl ether, tripropylene glycol methyl ether, propylene glycol n-butyl ether, propylene glycol n-propyl ether, propylene glycol monomethyl ether, diethylene glycol monomethyl ether and monoethyl ether, ethylene glycol 2-ethylhexyl ether, 3-phenyl-1-propanol, 2-phenyl-1-propanol, benzyl alcohol, benzyloxyethanol and phenoxyethanol, and mixtures thereof.

12. Composition according to any one of the preceding claims, **characterized in that** it comprises:

- one or more additional dyes, other than dyes of hydrophobic nature with a logP of greater than or equal to 2, chosen from natural dyes and nonnatural direct dyes, oxidation dye precursors, and combinations thereof, and/or
- one or more oxidizing agents, preferably hydrogen peroxide.

13. Dyeing process that consists in applying to keratin fibers, especially human keratin fibers such as the hair, a composition as defined in any one of the preceding claims

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2771286
- WO 9515144 A
- WO 9501772 A
- EP 714954 A
- FR 2189006
- FR 2285851
- FR 2140205
- EP 1378544 A
- EP 1674073 A
- GB 1026978 A
- GB 1153196 A
- FR 2801308
- DE 2359399
- JP 63169571 A
- JP 5063124 A
- EP 0770375 A
- WO 9615765 A
- DE 3843892
- DE 4133957
- WO 9408969 A
- WO 9408970 A
- FR 2733749 A
- DE 19543988
- FR 2886136
- EP 0173109 A


### Littérature non-brevet citée dans la description


- **MEYLAN ; HOWARD.** Atom / Fragment contribution method for estimating octanol-water partition coefficient. *J. Pharm. Sci.,* 1995, vol. 84, 83-92
- **CHARLES M.HANSEN.** Hansen solubility parameters A user's handbook. CRC Press, 2000, 167-185
- Handbook of Solubility Parameters and other cohesion parameters. CRC, Press, 95-121, 177-185
- Handbook of Solubility Parameters and other cohesion parameters. CRC Press, 95-121, 177-185
- **BAGDA, E.** The relation between surface tension and solubility parameter in liquids. *Farbe Lack,* 1978, vol. 84, 212
- **WALTER NOLL.** Chemistry and Technology of Silicones. Academic Press, 1968
- **G. FONNUM ; J. BAKKE ; FK. HANSEN.** *Colloid Polym. Sci,* 1993, vol. 271, 380.389
- *CHEMICAL ABSTRACTS,* 482-89-3
- *CHEMICAL ABSTRACTS,* 522-75-8
- *CHEMICAL ABSTRACTS,* 2379-74-0